# EUROPEAN PATENT SPECIFICATION

(11) **EP 3 932 490 B1**
(45) Date of publication and mention of the grant of the patent: **02.04.2025**
(21) Application number: 20762697.9
(22) Date of filing: 12.02.2020
(51) Int. Cl.: A61P 29/00, A61P 37/08, A61P 17/00, A61P 17/04, A61P 17/06, A61K 31/4178, A61K 31/519

(54) **ANTIPRURITIC AGENT USING PAC1 RECEPTOR ANTAGONIST**
ANTIPRURITISCHES MITTEL UNTER VERWENDUNG VON PAC1-REZEPTORANTAGONISTEN
AGENT ANTIPRURIGINEUX UTILISANT UN ANTAGONISTE DU RÉCEPTEUR PAC1

(30) Priority: 27.02.2019 JP 2019034313
(43) Date of publication of application: 05.01.2022
(73) Proprietor: Kagoshima University, Kagoshima-shi, Kagoshima 890-8580 (JP)
(72) Inventor: KURIHARA, Takashi, Kagoshima-shi, Kagoshima 890-8580 (JP); TAKASAKI, Ichiro, Toyama-shi, Toyama 930-8555 (JP); TOYOOKA, Naoki, Toyama-shi, Toyama 930-8555 (JP); GOUDA, Hiroaki, Tokyo 142-8555 (JP)
(74) Representative: J A Kemp LLP
(86) International application number: PCT/JP2020/005283
(87) International publication number: WO 2020/175134

(56) References cited:
- EP-A1- 3 689 870
- WO-A1-2017/029202
- WO-A1-2019/065794
- JP-A- 2004 224 775
- CHUQUILIN MIGUEL ET AL: "Neurocutaneous disease Cutaneous neuroanatomy and mechanisms of itch and pain", JOURNAL OF THE AMERICAN ACADEMY OF DERMATOLOGY, MOSBY, INC, US, vol. 74, no. 2, 15 January 2016 (2016-01-15), pages 197 - 212, XP029385830, ISSN: 0190-9622, DOI: 10.1016/J.JAAD.2015.04.060
- TAKASAKI ICHIRO, WATANABE AI, YOKAI MASAFUMI, WATANABE YURIE, HAYAKAWA DAICHI, NAGASHIMA RYOTA, FUKUCHI MAMORU, OKADA TAKUYA, TOYO: "In silico screening identified novel small-molecule antagonists of PAC1 receptor", J PHARMACOL EXP THER, vol. 365, no. 1, 2018, pages 1 - 8, XP055734734
- REICH A ET AL.: "Mediators of pruritus in psoriasis", MEDIATORS OF INFLAMMATION, vol. 2007, 2007, pages 1 - 6, XP055734740
- STEINHOFF, M ET AL.: "Identification of pituitary adenylate cyclase activating polypeptide (PACAP) and PACAP type 1 receptor in human skin: expression of PACAP-38 is increased in patients with psoriasis", REGUL PEPT, vol. 80, no. 1-2, 1999, pages 49 - 55, XP002552244, DOI: 10.1016/S0167-0115(99)00010-5
- REGLODI D ET AL.: "PACAP is an endogenous protective factor -insights from PACAP-deficient mice", J MOL NEUROSCI, vol. 48, no. 3, 2012, pages 482 - 492, XP035115161, DOI: 10.1007/s12031-012-9762-0

## Description

### Technical Field

The present invention relates to a composition comprising a PAC1 receptor antagonist, for use as an antipruritic agent.

### Background Art

Itch is one of cutaneous senses, which people experience on a daily basis. In recent years, since the number of patients with allergic diseases has been increased, and further, since aging of population has been sharply increased in advanced countries, it is expected that various pruritic diseases, including an itch associated with dry skin or an itch associated with systemic diseases such as kidney disease or liver disease, will be increased.

A first choice for the treatment of itch is an antihistamine. However, the antihistamine does not exhibit effects on an itch associated with skin diseases such as atopic dermatitis or psoriasis, and an itch associated with systemic diseases such as kidney disease or liver disease. For cutaneous pruritus associated with atopic dermatitis, topical steroids and tacrolimus topical drugs are used. Topical steroids provide striae cutis distensae and an increase in skin infections associated with weakened local immunity. Tacrolimus topical drugs provide local burning sensation, and it is said that approximately 30% of patients treated with the tacrolimus topical drugs cannot obtain sufficient effects. Such a patient group must be treated by short-term oral administration of steroid or cyclosporin. However, there is a concern regarding severe harmful action, and thus, steroid or cyclosporin cannot be used for a long period of time.

This year, a novel drug (dupilumab) has appeared to treat patients with high severity, on whom the treatment with topical steroids/tacrolimus topical drugs does not exhibit sufficient effects. This drug is a human type anti-human IL-4/IL-13 receptor monoclonal antibody, and it is said that this drug is effective for approximately 40% of patients with high severity. Since this drug is a biopharmaceutical product, it is expensive (patient's self-pay burden: several tens of thousands of yen/month). Regarding psoriasis as well, a biopharmaceutical product (human type human IL-17 receptor A monoclonal antibody) has been recently introduced, but this biopharmaceutical product is also an expensive therapeutic agent.

For cutaneous pruritus developed in dialysis patients with renal failure or patients with liver failure, a κ-opioid receptor agonist, nalfurafine, has been used since 2009, and this drug exhibits a high response rate (i.e., this drug is effective for 70% of the patients). On the other hand, it is said that approximately 30% of the patients could not obtain sufficient effects from this drug.

Accordingly, it has been still desired to develop a novel antipruritic agent that has an action mechanism different from those of the aforementioned drugs and can be utilized at low costs.

PACAP (Pituitary Adenylate Cyclase-Activating Polypeptide) is a neuropeptide which was originally isolated from ovine hypothalamus based on its ability to stimulate adenylate cyclase in rat anterior pituitary cell cultures and structurally determined in 1989, and causes mechanical pain hypersensitivity (mechanical allodynia: the phenomenon of feeling pain even though the patient is only touched) through spinal PAC1 receptors (Non Patent Literature 1). However, it is unclear what type of pain PACAP is involved in clinically (in a human).

It is suggested that PACAP is involved in peripheral neuropathic (spinal neuropathic) pain (SNL model) at the level of animal experiments (mice and rats) (Non Patent Literature 2). However, it is unclear which PACAP receptor is involved (PACAP receptors have at least three types, which are PAC1, VPAC1 and VPAC2).

Patent Literature 1 describes that PACAP has a sweat secretion-promoting action and is useful as a preventive or therapeutic drug for dry skin.

Non Patent Literature 3 shows the structures of the compounds PA-8 and PA-9, which were selected from the existing compound database in which 4 million or more items are registered, and also shows that these compounds have PAC1 receptor antagonistic action and analgesic action. However, the antipruritic action thereof is not examined.

The compound PA-8 is a compound represented by the following formula (A): 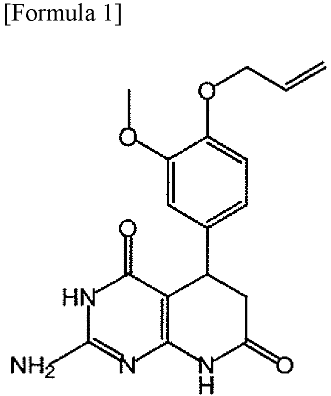 The compound PA-9 is a compound represented by the following formula (B):

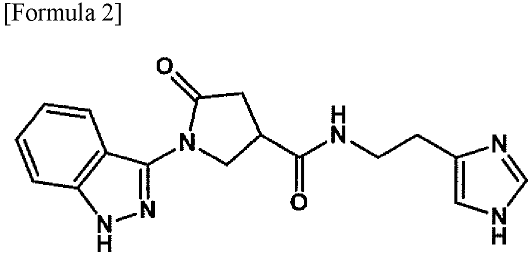

These compounds are common in that the compounds each have a nitrogen-containing heterocyclic structure containing two or more nitrogen atoms and a lactam structure.

Although Non Patent Literatures 4 and 5 describe a pyrido[2,3-d]pyrimidine-4,7-dione derivative represented by the following formula (C): 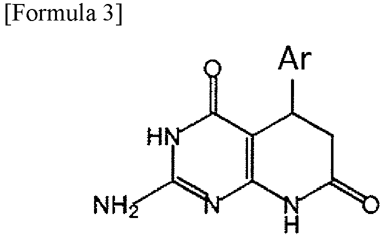 wherein Ar is a substituted phenyl group and a method of synthesizing the same, Non Patent Literatures 4 and 5 do not mention PAC1 receptor antagonistic action and antipruritic action.

### Citation List

### Patent Literature

[Patent Literature 1] JP 2016-169205 A

### Non Patent Literature

[Non Patent Literature 1] Yokai et al. Mol. Pain 2016. 12, 1-13.
[Non Patent Literature 2] Mabuchi et al., J Neurosci 2004, 24, 7283-7291.
[Non Patent Literature 3] Takasaki et al. J. Pharmacol. Exp. Ther. 2018, 365, 1-8.
[Non Patent Literature 4] Shi, D. Q. et al. J. Heterocyclic Chem. 2009, 46, 1331-1334
[Non Patent Literature 5] Tu, S. et al., Bioorg. Med. Chem. Lett. 2006, 16, 3578-3581

### Summary of Invention

The present invention is defined in the appended claims. Embodiments not falling within the scope of the appended claims do not form part of the invention. Any references in the description to methods of treatment refer to the compounds, pharmaceutical compositions and medicaments of the present invention for use in a method for treatment of the human (or animal) body by therapy.

### Technical Problem

An object of the present invention is to provide a novel antipruritic agent having an action mechanism different from those of conventional antipruritic agents.

### Solution to Problem

In order to achieve the aforementioned object, the present inventors have found a specific compound having excellent antipruritic effects from among PAC1 receptor antagonist candidate compounds, thereby completing the present invention.

More specifically, the present invention will be summarized as follows.
(1) A composition comprising a compound represented by the following formula (I), 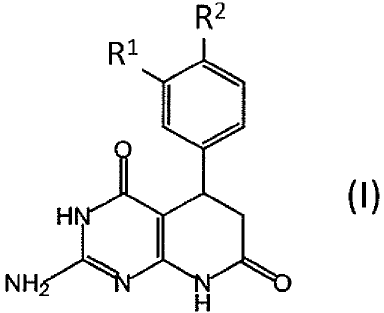
   wherein R¹ is a hydrogen atom, a C₁₋₆-alkyl group, a C₁₋₆-alkoxy group, a C₂₋₆-alkenyloxy group, a halogen atom, a C₁₋₆-haloalkyl group, a C₁₋₆-haloalkoxy group, or a substituted or unsubstituted phenyl group; and R² is a hydrogen atom, a C₁₋₆-alkyl group, a C₁₋₆-alkoxy group, a C₂₋₆-alkenyloxy group, a halogen atom, a C₁₋₆-haloalkyl group, a C₁₋₆-haloalkoxy group, or a substituted or unsubstituted phenyl group;
   or a salt thereof, or a solvate thereof,
   for use as an antipruritic agent.
(2) The composition according to (1) for use in treating and/or preventing itch associated with contact dermatitis, atopic dermatitis, dry skin and/or psoriasis.
(3) A composition comprising a compound represented by the following formula (II), 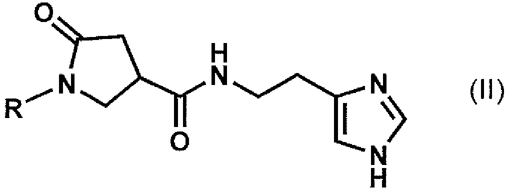
   wherein R is an indazolyl group substituted with a halogen atom; a substituted or unsubstituted phenyl group; a pyrazolyl group; or a substituted or unsubstituted aralkyl group;
   or a salt thereof, or a solvate thereof,
   for use as an antipruritic agent.
(4) The composition for use according to (3), wherein R is an indazolyl group substituted with a halogen atom in the formula (II).
(5) The composition for use according to (3), wherein R is an indazolyl group substituted with a chlorine atom in the formula (II).
(6) The composition according to any one of (3) to (5) for use in treating and/or preventing itch associated with contact dermatitis, atopic dermatitis, dry skin and/or psoriasis.

### Advantageous Effects of Invention

The composition for use according to the present invention comprises a PAC1 receptor antagonist as an active ingredient, and has an action mechanism different from those of conventional antipruritic agents such as an antihistamine, a steroid and an immunosuppressant. In addition, the present composition can avoid a harmful action found in such a steroid or an immunosuppressant.

Moreover, since the composition of the present invention has a point of action in both the peripheral (intradermal) nervous system and the central (dorsal horn of spinal cord) nervous system, it is highly effective and thus, it can reduce the necessity of the combined use of multiple drugs. Hence, the present composition can avoid of an unexpected appearance of harmful action that is attended with the combined use of multiple drugs.

It is said that 80% or more of elderly people have dry skin, and that a half thereof suffers from itch. As such, an antipruritic agent is highly required for dry skin in the super-aging society in Japan. It can be expected that the composition of the present invention will improve the quality of life (QOL) of elderly people suffering from the itch caused by dry skin.

### Brief Description of Drawings

[Figure 1] Figure 1 shows the evaluation results of a behavioral experiment using the serotonin (5-HT)-induced itch mouse model.
[Figure 2] Figure 2 shows an outline of the procedures of a behavioral experiment using the dry skin itch mouse model.
[Figure 3] Figure 3 shows the evaluation results of a behavioral experiment using the dry skin itch mouse model.
[Figure 4] Figure 4 shows the evaluation of PA-8 and derivatives thereof (compound 2j and compound 2o) using PACAP receptor-expressing cells.
[Figure 5] Figure 5 shows the evaluation of PA-9 and a derivative thereof (compound 3d) using PACAP receptor-expressing cells.
[Figure 6] Figure 6 shows that an itch behavior is caused by intradermal administration of PACAP.
[Figure 7] Figure 7 shows that an itch behavior induced by PACAP intradermal administration is suppressed by simultaneous intradermal administration of PA-8 with PACAP or by subcutaneous pre-administration of an opioid receptor antagonist, naltrexone (NTX).
[Figure 8] Figure 8 shows that an itch behavior induced by PACAP intradermal administration is suppressed in a dose-dependent manner by simultaneous intradermal administration of PA-8 or compound 2o with PACAP.
[Figure 9] Figure 9 shows that an itch behavior induced by PACAP intradermal administration is suppressed in a dose-dependent manner by simultaneous intradermal administration of PA-9 or compound 3d with PACAP.
[Figure 10] Figure 10 shows that an itch behavior induced by chloroquine intradermal administration is suppressed by intrathecal pre-administration of PA-8 or PA-9, but is not suppressed by simultaneous intradermal administration of PA-8 or PA-9 with PACAP.
[Figure 11] Figure 11 shows that an itch behavior induced by Compound 48/80 intradermal administration is suppressed by intrathecal pre-administration of PA-8 or PA-9, but is not suppressed by simultaneous intradermal administration of PA-8 or PA-9 with PACAP.
[Figure 12] Figure 12 shows an outline of the procedures of a behavioral experiment using the atopic dermatitis mouse model.
[Figure 13] Figure 13 shows the results obtained by evaluating the time spent in scratching of each group in a behavioral experiment using the atopic dermatitis mouse model.
[Figure 14] Figure 14 shows the results obtained by evaluating the time spent in scratching of each group in a behavioral experiment using the psoriasis mouse model.

### Description of Embodiments

The present invention will be described in detail hereinafter.

Examples of the C₁₋₆-alkyl group represented by R¹ or R² in the formula (I) include a methyl group, an ethyl group, a propyl group, an isopropyl group, a butyl group, an isobutyl group, a sec-butyl group (1-methylpropyl group), a tert-butyl group, a pentyl group, an isopentyl group, 1-ethylpropyl group, a hexyl group, a cyclopropyl group, a cyclobutyl group, a cyclopentyl group and a cyclohexyl group.

Examples of the C₁₋₆-alkoxy group represented by R¹ or R² in the formula (I) include a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopropyloxy group, a cyclobutyloxy group, a cyclopentyloxy group and a cyclohexyloxy group.

Examples of the C₂₋₆-alkenyloxy group represented by R¹ or R² in the formula (I) include a vinyloxy group, a 1-propenyloxy group, an allyloxy group, a 1-butenyloxy group, a 2-butenyloxy (crotyloxy) group, a pentenyloxy group, a 3-methyl-2-butenyloxy (prenyloxy) group and a hexenyloxy group.

Examples of the halogen atom represented by R¹ or R² in the formula (I) include a fluorine atom, a chlorine atom, a bromine atom and an iodine atom.

Examples of the C₁₋₆-haloalkyl group represented by R¹ or R² in the formula (I) include a trifluoromethyl group.

Examples of the C₁₋₆-haloalkoxy group represented by R¹ or R² in the formula (I) include a trifluoromethoxy group.

The phenyl group represented by R¹ or R² in the formula (I) may be substituted with one or more substituents selected from a C₁₋₆-alkyl group, a C₁₋₆-alkoxy group, a methylenedioxy group, a C₂₋₆-alkenyloxy group, an aralkyloxy group (for example, a benzyloxy group, a 4-methylbenzyloxy group, a 3-methylbenzyloxy group, a 2-methylbenzyloxy group, a 4-fluorobenzyloxy group, a 3-fluorobenzyloxy group, a 4-chlorobenzyloxy group and a 3-chlorobenzyloxy group), a halogen atom, a C₁₋₆-haroalkyl group, a C₁₋₆-haroalkoxy group, a substituted or unsubstituted phenyl group, an acyl group (for example, a C₁₋₆-aliphatic acyl group such as a formyl group, an acetyl group, a propanoyl group, a butanoyl group, a pentanoyl group and a hexanoyl group; and an aroyl group such as a benzoyl group and a toluoyl group), an acyloxy group (for example, a C₁₋₆-aliphatic acyloxy group such as a formyloxy group, an acetoxy group, a propanoyloxy group, a butanoyloxy group, a pentanoyloxy group and a hexanoyloxy group; and an aroyloxy group such as a enzoyloxy group and a toluoyloxy group), a hydroxyl group, a carboxyl group, an acetamido group, a carbamoyl group, a cyano group, a nitro group, and the like.

Among the compounds represented by the formula (I), compounds wherein R¹ is a C₁₋₆-alkoxy group or a C₁₋₆-haroalkoxy group (for example, a trifluoromethoxy group) are preferable.

An indazolyl group substituted with a halogen atom and represented by R in the formula (II) is not particularly limited as long as it is an indazolyl group substituted with at least one halogen atom selected from fluorine atoms, chlorine atoms, bromine atoms and iodine atoms (preferably chlorine atom). However, preferable examples thereof include a 3-indazolyl group substituted with at least one chlorine atom.

Examples of the aralkyl group represented by R in the formula (II) include a benzyl group and a phenethyl group.

The phenyl and aralkyl group represented by R in the formula (II) may be substituted with one or more substituents selected from a C₁₋₆-alkyl group (for example, a methyl group, an ethyl group, a propyl group, an isopropyl group, a butyl group, an isobutyl group, a sec-butyl group (1-methylpropyl group), a tert-butyl group, a pentyl group, an isopentyl group, 1-ethylpropyl group, a hexyl group, a cyclopropyl group, a cyclobutyl group, a cyclopentyl group and a cyclohexyl group), a C₁₋₆-alkoxy group (for example, a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopropyloxy group, a cyclobutyloxy group, a cyclopentyloxy group and a cyclohexyloxy group), a methylenedioxy group, a C₂₋₆-alkenyloxy group, an aralkyloxy group (for example, a benzyloxy group, a 4-methylbenzyloxy group, a 3-methylbenzyloxy group, a 2-methylbenzyloxy group, a 4-fluorobenzyloxy group, a 3-fluorobenzyloxy group, a 4-chlorobenzyloxy group and a 3-chlorobenzyloxy group), a halogen atom (such as a fluorine atom, a chlorine atom, a bromine atom and an iodine atom), a C₁₋₆-haroalkyl group, a C₁₋₆-haroalkoxy group, a substituted or unsubstituted phenyl group, an acyl group (for example, a C₁₋₆-aliphatic acyl group such as a formyl group, an acetyl group, a propanoyl group, a butanoyl group, a pentanoyl group and a hexanoyl group; and an aroyl group such as a benzoyl group and a toluoyl group), an acyloxy group (for example, a C₁₋₆-aliphatic acyloxy group such as a formyloxy group, an acetoxy group, a propanoyloxy group, a butanoyloxy group, a pentanoyloxy group and a hexanoyloxy group; and an aroyloxy group such as a benzoyloxy group and a toluoyloxy group), a hydroxyl group, a carboxyl group, an acetamido group, a carbamoyl group, a cyano group, a nitro group, and the like.

The salt of a compound represented by the formula (I) or (II) is preferably a pharmaceutically acceptable salt, and examples of the salt include a salt of the compound with an inorganic acid such as hydrochloric acid, sulfuric acid, phosphoric acid, hydrobromic acid, hydroiodic acid, nitric acid, pyrosulfuric acid and metaphosphoric acid or with an organic acid such as citric acid, benzoic acid, acetic acid, propionic acid, fumaric acid, maleic acid and sulfonic acid (for example, methanesulfonic acid, p-toluene sulfonic acid, naphthalene sulfonic acid).

Examples of a solvate of a compound represented by the formula (I) or (II), or a salt thereof include a hydrate.

A compound represented by the formula (I) can be produced, for example, according to the method described in Shi, D. Q. et al. J. Heterocyclic Chem. 2009, 46, 1331-1334, or Tu, S. et al. Bioorg. Med. Chem. Lett. 2006, 16, 3578-3581, as follows.

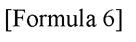

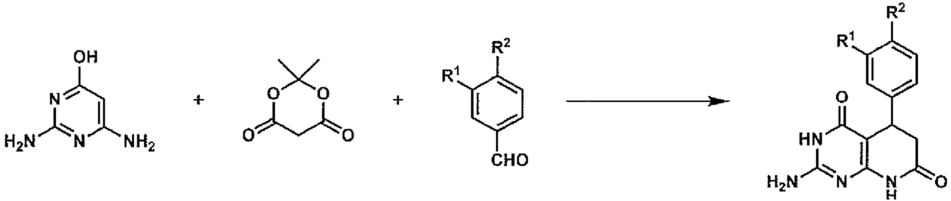

wherein R¹ and R² have the same meaning as defined in the formula (I).

Specifically, a target compound (I) can be manufactured by heating and reacting the corresponding aromatic aldehyde compound, 2,4-diamino-6-hydroxypyrimidine (another name: 2,6-diaminopyrimidin-4(3H)-one) and Meldrum's acid, (i) in water in the presence of triethylbenzylammonium chloride, (ii) while being irradiated with a microwave, or (iii) in an organic solvent.

A compound represented by the formula (II) can be produced, for example, according to the method described in JP 2006-510596 A, as follows. 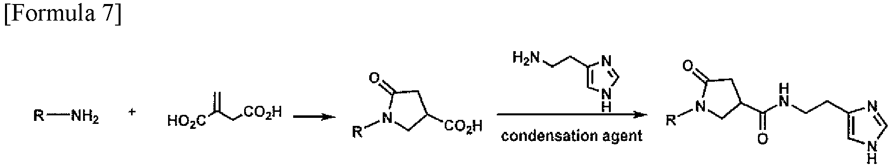 wherein R has the same meaning as defined in the formula (II).

Specifically, a target compound (II) can be manufactured by reacting the corresponding amine compound and itaconic acid to convert them into γ-lactam carboxylic acid, and then reacting the γ-lactam carboxylic acid with histamine in the presence of a condensing agent (for example, carbodiimide).

The product obtained as mentioned above may be purified by a customary method, for example, column chromatography using, e.g., silica gel, as a carrier and a recrystallization method using, e.g., methanol, ethanol, chloroform, dimethyl sulfoxide, n-hexane-ethyl acetate or water. Examples of an elution solvent for column chromatography include methanol, ethanol, chloroform, acetone, hexane, dichloromethane, ethyl acetate and mixed solvents of these.

The compound as mentioned above can be used as an antipruritic agent in combination with a customary pharmaceutical carrier. The dosage form thereof is not particularly limited and appropriately selected and used depending on needs. Examples of the dosage form include oral agents such as a tablet, a capsule, a granule, a fine granule, a powder, a sustained release preparation, a liquid preparation, a suspension, an emulsion, a syrup and an elixir and parenteral agents such as an injection and a suppository.

An oral agent is produced by using, for example, starch, lactose, sucrose, mannitol, carboxymethylcellulose and inorganic salts in accordance with an ordinary method. In addition to these components, e.g., a binder, a disintegrant, a surfactant, a lubricant, a glidant, a flavoring agent, a colorant and/or a perfume can be appropriately added.

Examples of the binder include starch, dextrin, gum arabic, gelatin, hydroxypropyl starch, methylcellulose, sodium carboxymethylcellulose, hydroxypropylcellulose, crystalline cellulose, ethylcellulose, polyvinyl pyrrolidone and macrogol.

Examples of the disintegrant include starch, hydroxypropyl starch, sodium carboxymethylcellulose, calcium carboxymethylcellulose, carboxymethylcellulose and a low-substituted hydroxypropylcellulose.

Examples of the surfactant include sodium lauryl sulfate, soy lecithin, sucrose fatty acid ester and polysorbate 80.

Examples of the lubricant include talc, wax, hydrogenated vegetable oil, sucrose fatty acid ester, magnesium stearate, calcium stearate, aluminum stearate and polyethylene glycol.

Examples of the glidant include light anhydrous silicic acid, dry aluminum hydroxide gel, synthetic aluminum silicate and magnesium silicate.

An injection is produced in accordance with an ordinary method. As a diluent, generally, distilled water for injection, saline, a glucose solution, olive oil, sesame oil, peanut oil, soybean oil, corn oil, propylene glycol, polyethylene glycol, and/or the like can be used. If necessary, a disinfectant, a preservative, a stabilizer, an isotonic agent, a soothing agent, and/or the like may be added. In view of stability, an injection can be added in, e.g., a vial, frozen and subjected to ordinary lyophilization to remove a water content. From the lyophilized injection, a liquid preparation can be prepared again immediately before use. The content of a compound of the formula (I) or (II) in the injection may be varied between the 5 and 50 wt%; however, the content is not limited to this.

Examples of other parenteral agents include a suppository for intrarectal administration and an external preparation for local administration (for example, eye drop, ointment and paste), which can be produced in accordance with an ordinary method.

The administration schedule of an antipruritic agent formulated varies depending on, e.g., the dosage form and the route of administration, and, for example, can be administered once to four times per day in a period from a week to 3 months.

In order to obtain a desired effect, the dose of an oral agent, which varies depending on the age, body weight and severity of a disease of a patient, is usually, for example, 0.1 to 1000 mg and preferably 1 to 500 mg per adult in terms of the weight of a compound of the formula (I) or (II), and suitably divided into several portions per day and administered.

In order to obtain a desired effect, the dose of a parenteral agent, which varies depending on the age, body weight and severity of a disease of a patient, is usually, for example, 0.1 to 1000 mg and preferably 1 to 500 mg per adult in terms of the weight of a compound of the formula (I) or (II), and suitably administered by intravenous injection, intravenous drip infusion, subcutaneous injection, intramuscular injection, intraperitoneal administration or intrathecal administration.

The composition according to the present invention can be used for treating and/or preventing itch associated with dry skin disease such as dry skin and xeroderma; allergic dermatitis such as contact dermatitis and atopic dermatitis; psoriasis, insect bite and sting, grass rash, tinea pedis, and tinea corporis.

### Examples

Now, the present invention will be more specifically described below by way of examples.

### [Example 1] Synthesis of pyrido[2,3-d]pyrimidine derivatives

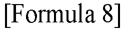

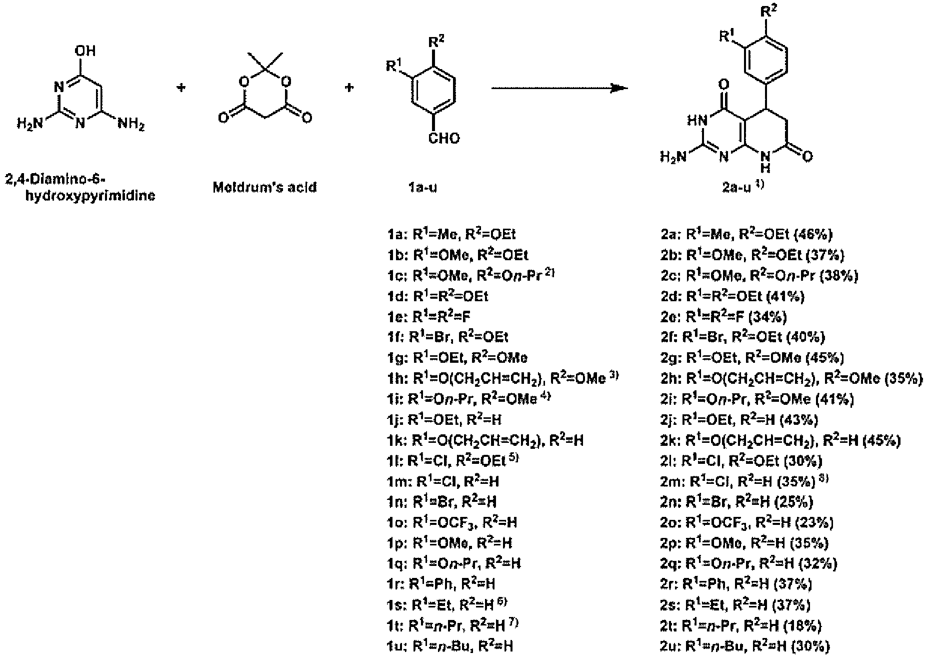

To a solution of the corresponding aldehyde (1.00 mmol) from the commercial aldehydes (1a, b, d, e, f, g, j, k, m, n, o, p, q and r) and the aldehydes known in Literatures (1c, h, i, l, s and t) in ethylene glycol (0.5 mL) were sequentially added at room temperature 2,4-diamino-6-hydroxypyrimidine (0.67 mmol) and Meldrum's acid (1.00 mmol), and the mixture was stirred at 100°C for 20 hours in an Ar atmosphere in accordance with a method described in Literature ¹⁾. The reaction liquid was let to cool, then filtered, and further washed with methanol (0.5 mL × 3) to obtain pale yellow crystals 2a to u, respectively.

### 2-Amino-5-(4-ethoxy-3-methylphenyl)-5,8-dihydro-3H,6H-pyrido[2,3-d]pyrimidine-4,7-dione (2a)

Yield: 46%; mp: >300 °C; IR (KBr): 3455, 3166, 2856, 2699, 1578, 1477 cm⁻¹; ¹H NMR (400 MHz, DMSO-d6): δ 10.55 (1H, br s), 10.01 (1H, s), 6.90 (1H, d, J = 2.4 Hz), 6.84 (1H, dd, J = 8.8, 2.4 Hz), 6.77 (1H, d, J = 8.8 Hz), 6.51 (2H, br s), 4.00 (1H, d, J = 7.6 Hz), 3.95 (2H, q, J = 7.0 Hz), 2.88 (1H, dd, J = 16.0, 7.6 Hz), 2.42 (1H, d, J = 16.0 Hz), 2.07 (3H, s), 1.29 (3H, t, J = 7.0 Hz); ¹³C NMR (100 MHz, DMSO-d6): δ 171.10, 161.42, 156.45, 155.20, 154.95, 135.08, 128.74, 125.49, 124.61, 111.22, 92.07, 63.14, 32.12, 16.21, 14.83; MS (EI) m/z 314 (M+).

### 2-Amino-5-(4-ethoxy-3-methoxyphenyl)-5,8-dihydro-3H,6H-pyrido[2,3-d]pyrimidine-4,7-dione (2b)

Yield: 37%; mp: >300 °C; IR (KBr): 3462, 3309, 2850, 2743, 1582, 1521 cm⁻¹; ¹H NMR (400 MHz, DMSO-d6): δ 10.57 (1H, br s), 10.02 (1H, s), 6.84 (1H, d, J = 2.3 Hz), 6.78 (1H, d, J = 8.7 Hz), 6.53 (1H, dd, J = 8.7, 2.3 Hz), 6.50 (2H, br s), 4.05 (1H, d, J = 7.7 Hz), 3.92 (2H, q, J = 6.9 Hz), 3.69 (3H, s), 2.88 (1H, dd, J = 16.4, 7.7 Hz), 1.27 (3H, t, J = 6.9 Hz); ¹³C NMR (100 MHz, DMSO-d6): δ 171.34, 161.47, 156.43, 155.01, 148.89, 146.63, 136.09, 117.65, 112.90, 111.24, 92.03, 63.73, 55.38, 38.71, 32.44, 14.81; MS (EI) m/z 330 (M+).

### 2-Amino-5-(3-methoxy-4-propoxyphenyl)-5,8-dihydro-3H,6H-pyrido[2,3-d]pyrimidine-4,7-dione (2c)

Yield: 38%; mp: >300 °C; IR (KBr): 3450, 3167, 2959, 2876, 1652, 1591 cm⁻¹; ¹H NMR (400 MHz, DMSO-d6): δ 10.57 (1H, br s), 10.03 (1H, br s), 6.83 (1H, d, J = 2.3 Hz), 6.79 (1H, d, J = 8.4 Hz), 6.52 (1H, dd, J = 8.4, 2.3 Hz), 6.51 (2H, br s), 4.05 (1H, d, J = 7.9 Hz), 3.82 (2H, t, J = 7.1 Hz), 3.69 (3H, s), 2.88 (1H, dd, J = 16.4, 7.9 Hz), 1.67 (2H, sext, J = 7.1 Hz), 0.92 (3H, t, J = 7.1 Hz); ¹³C NMR (100 MHz, DMSO-d6): δ 171.22, 161.69, 158.44, 157.50, 146.831, 145.48, 137.33, 117.79, 113.05, 111.44, 99.65, 69.86, 55.57, 38.73, 32.52, 22.20, 10.53; MS (EI) m/z 344 (M+).

### 2-Amino-5-(3,4-diethoxyphenyl)-5,8-dihydro-3H,6H-pyrido[2,3-d]pyrimidine-4,7-dione (2d)

Yield: 41%; mp: >300 °C; IR (KBr): 3188, 2977, 2868, 1653, 1635 cm⁻¹; ¹H NMR (400 MHz, DMSO-d6): δ 10.57 (1H, s), 10.01 (1H, s), 6.81 (1H, d, J = 1.8 Hz), 6.78 (1H, d, J = 8.8 Hz), 6.53 (1H, dd, J = 8.8, 1.8 Hz), 6.50 (2H, br s), 4.03 (1H, d, J = 7.8 Hz), 3.93 (2H, q, J = 6.9 Hz), 3.92 (2H, q, J = 6.9 Hz), 2.87 (1H, dd, J = 16.0, 7.8 Hz), 1.28 (3H, t, J = 6.9 Hz), 1.26 (3H, t, J = 6.9 Hz); ¹³C NMR (100 MHz, DMSO-d6): δ 171.27, 161.47, 156.42, 154.97, 148.11, 146.87, 136.19, 117.94, 113.45, 112.69, 92.07, 63.83, 63.78, 38.66, 32.37, 14.82; MS (EI) m/z 344 (M+).

### 2-Amino-5-(3,4-difluorophenyl)-5,8-dihydro-3H,6H-pyrido[2,3-d]pyrimidine-4,7-dione (2e)

Yield: 34%; mp: >300 °C; IR (KBr): 3471, 3161, 1646, 1592 cm⁻¹; ¹H-NMR (400 MHz, DMSO-d6): δ 10.62 (1H, br s), 10.13 (1H, br s), 7.32 (1H, dt, J = 10.8, 8.4 Hz), 7.18 (1H, ddd, J = 10.8, 8.4, 2.4 Hz), 6.95 (1H, m), 6.57 (2H, br s), 4.12 (1H, d, J = 7.2 Hz), 2.94 (1H, dd, J = 16.3, 7.2 Hz); ¹³C NMR (125 MHz, DMSO-d6): δ 170.86, 161.41, 156.68, 155.24, 149.02, 147.17, 141.45, 122.88, 117.41 (d, J = 17.0 Hz), 115.62 (d, J = 17.0 Hz), 91.05, 38.22, 32.24; MS (EI) m/z 292 (M+).

### 2-Amino-5-(3-bromo-4-ethoxyphenyl)-5,8-dihydro-3H,6H-pyrido[2,3-d]pyrimidine-4,7-dione (2f)

Yield: 40%; mp: >300 °C; IR (KBr): 3458, 3080, 2863, 2751, 1540, 1475 cm⁻¹; ¹H NMR (400 MHz, DMSO-d6): δ 10.57 (1H, br s), 10.09 (1H, s), 7.30 (1H, d, J = 2.4 Hz), 7.06 (1H, dd, J = 8.0, 2.4 Hz), 6.99 (1H, d, J = 8.0 Hz), 6.56 (2H, br s), 4.09 (1H, m), 4.04 (2H, q, J = 7.1 Hz), 2.90 (1H, dd, J = 16.2, 6.7 Hz), 1.31 (3H, t, J = 7.1 Hz); ¹³C NMR (100 MHz, DMSO-d6): δ 170.93, 161.39, 156.55, 155.10, 153.29, 137.33, 130.87, 126.80, 113.77, 110.89, 91.53, 64.39, 38.47, 31.82, 14.58; MS (EI) m/z 379 (M+).

### 2-Amino-5-(3-ethoxy-4-methoxyphenyl)-5,8-dihydro-3H,6H-pyrido[2,3-d]pyrimidine-4,7-dione (2g)

Yield: 45%; mp: >300 °C; IR (KBr): 3461, 3160, 2841, 1591, 1516 cm⁻¹; ¹H NMR (400 MHz, DMSO-d6): δ 10.58 (1H, br s), 10.03 (1H, s), 6.82 (1H, d, J = 2.4 Hz), 6.80 (1H, d, J = 8.4 Hz), 6.55 (1H, dd, J = 8.4, 2.4 Hz), 6.50 (1H, br s), 4.04 (1H, d, J = 7.8 Hz), 3.96-3.88 (2H, m), 3.68 (3H, s), 2.87 (1H, dd, J = 16.0, 7.8 Hz), 1.28 (3H, t, J = 7.2 Hz); ¹³C NMR (100 MHz, DMSO-d6): δ 171.30, 161.55, 156.51, 155.00, 147.88, 147.66, 136.10, 117.81, 112.20, 111.90, 92.11, 63.68, 55.54, 38.72, 32.42, 14.83; MS (EI) m/z 330 (M+).

### 5-(3-Allyloxy-4-methoxyphenyl)-2-amino-5,8-dihydro-3H,6H-pyrido[2,3-d]pyrimidine-4,7-dione (2h)

Yield: 35%; mp: >300 °C; IR (KBr): 3462, 3169, 1636, 1617, 1591 cm⁻¹; ¹H NMR (400 MHz, DMSO-d6): δ 10.58 (1H, br s), 10.02 (1H, s), 6.84 (1H, d, J = 2.0 Hz), 6.81 (1H, d, J = 8.4 Hz), 6.57 (1H, dd, J = 8.4, 2.0 Hz), 5.99 (1H, ddd, J = 17.6, 10.4, 5.2 Hz), 5.36 (1H, dd, J = 17.6, 2.0 Hz), 5.22 (1H, dd, J = 10.4, 2.0 Hz), 4.46 (2H, d, J = 5.2 Hz), 4.03 (1H, d, J = 7.2 Hz), 3.68 (3H, s), 2.87 (1H, dd, J = 15.8, 7.2 Hz); ¹³C NMR (100 MHz, DMSO-d6): δ 171.16, 158.72, 156.11, 154.99, 147.79, 147.55, 136.10, 133.95, 118.27, 117.88, 112.81, 112.09, 92.02, 69.10, 55.65, 32.41; MS (EI) m/z 342 (M+).

### 2-Amino-5-(4-methoxy-3-propoxyphenyl)-5,8-dihydro-3H,6H-pyrido[2,3-d]pyrimidine-4,7-dione (2i)

Yield: 41%; mp: >300 °C; IR (KBr): 3463, 3160, 2846, 1695, 1591, 1539, 1516 cm⁻¹; ¹H NMR (400 MHz, DMSO-d6): δ 10.58 (1H, br s), 10.03 (1H, s), 6.82 (1H, d, J = 2.4 Hz), 6.80 (1H, dd, J = 8.4, 2.4 Hz), 6.55 (1H, d, J = 8.4 Hz), 6.51 (2H, br s), 4.04 (1H, d, J = 7.2 Hz), 3.84-3.81 (2H, m), 3.67 (3H, s), 2.87 (1H, dd, J = 16.4, 7.2 Hz), 1.68 (2H, sext, J = 6.8 Hz), 0.94 (3H, t, J = 6.8 Hz); ¹³C NMR (100 MHz, DMSO-d6): δ 171.24, 161.48, 156.43, 154.96, 148.04, 147.68, 136.14, 117.78, 112.27, 112.04, 92.06, 69.63, 55.61, 38.66, 32.38, 22.12, 10.46; MS (EI) m/z 344 (M+).

### 2-Amino-5-(3-ethoxyphenyl)-5,8-dihydro-3H,6H-pyrido[2,3-d]pyrimidine-4,7-dione (2j):

Yield: 43%; mp: >300 °C; IR (KBr): 3447, 3170, 2854, 1592, 1539 cm⁻¹; ¹H NMR (400 MHz, DMSO-d6): δ 10.58 (1H, br s), 10.06 (1H, s), 7.15 (1H, t, J = 7.8 Hz), 6.72 (1H, dd, J = 7.8, 2.4 Hz), 6.70 (1H, dd, J = 7.8, 2.4 Hz), 6.66 (1H, d, J = 2.4 Hz), 6.53 (2H, br s), 4.07 (1H, d, J = 7.4 Hz), 3.94 (2H, q, J = 6.0 Hz), 2.91 (1H, dd, J = 16.3, 7.4 Hz), 1.28 (3H, t, J = 6.0 Hz); ¹³C NMR (100 MHz, DMSO-d6): δ 171.04, 161.40, 158.57, 156.59, 155.03, 145.32, 129.28, 118.56, 113.19, 111.58, 91.65, 62.78, 38.50, 32.86, 14.65; MS (EI) m/z 300 (M+).

### 5-(3-Allyloxyphenyl)-2-amino-5,8-dihydro-3H,6H-pyrido[2,3-d]pyrimidine-4,7-dione (2k)

Yield: 45%; mp: >300 °C; IR (KBr): 3085, 2855, 2727, 1520 cm⁻¹; ¹H NMR (400 MHz, DMSO-d6): δ 10.58 (1H, br s), 10.06 (1H, s), 7.15 (1H, t, J = 7.6 Hz), 6.76 (1H, dd, J = 7.6, 2.4 Hz), 6.71 (2H, m), 6.53 (2H, br s), 6.00 (1H, ddd, J = 17.4, 10.6, 5.5 Hz), 5.36 (1H, dd, J = 17.4, 1.6 Hz), 5.23 (1H, dd, J = 10.6, 1.6 Hz), 4.48 (2H, d, J = 5.5 Hz), 4.07 (1H, d, J = 7.5 Hz), 3.89 (1H, dd, J = 16.2, 7.5 Hz); ¹³C NMR (100 MHz, DMSO-d6): δ 171.09, 161.41, 158.26, 156.58, 155.06, 145.35, 133.75, 129.48, 118.78, 117.56, 113.43, 112.03, 91.60, 68.11, 38.50, 32.86; MS (EI) m/z 312 (M+).

### 2-Amino-5-(3-chloro-4-ethoxyphenyl)-5,8-dihydro-3H,6H-pyrido[2,3-d]pyrimidine-4,7-dione (2l)

Yield: 30%; mp: >300 °C; ¹H NMR (400 MHz, DMSO-d6): δ 10.60 (1H, br s), 10.09 (1H, s), 7.15 (1H, s), 7.02 (2H, s), 6.55 (2H, br s), 4.06 (1H, d, J = 8.0 Hz), 4.05 (2H, q, J = 7.0 Hz), 2.91 (1H, dd, J = 16.0, 8.0 Hz), 1.31 (3H, t, J = 7.0 Hz); ¹³C NMR (100 MHz, DMSO-d6): δ 171.0, 161.38, 156.52, 155.11, 153.38, 136.81, 127.90, 126.09, 121.07, 113.90, 91.53, 64.28, 38.42, 31.87, 14.57.

### 2-Amino-5-(3-chlorophenyl)-5,8-dihydro-3H,6H-pyrido[2,3-d]pyrimidine-4,7-dione (2m)

Yield: 35%; mp: >300 °C; ¹H NMR (400 MHz, DMSO-d6): δ 10.61 (1H, br s), 10.11 (1H, s), 7.30 (1H, t, J = 8.0 Hz), 7.24 (1H, d, J = 8.0 Hz), 7.16 (1H, s), 7.11 (1H, d, J = 8.0 Hz), 6.57 (2H, br s), 4.13 (1H, d, J = 8.0 Hz), 2.95 (1H, dd, J = 16.0, 8.0 Hz); ¹³C NMR (100 MHz, DMSO-d6): δ 170.85, 161.42, 156.76, 155.18, 146.35, 133.07, 130.46, 126.49, 126.41, 125.20, 91.61, 38.24, 32.72.

### 2-Amino-5-(3-bromophenyl)-5,8-dihydro-3H,6H-pyrido[2,3-d]pyrimidine-4,7-dione (2n)

Yield: 25%; mp: >300 °C; ¹H NMR (400 MHz, DMSO-d6): δ 10.61 (1H, br s), 10.12 (1H, s), 7.38 (1H, d, J = 8.0, 1.8 Hz), 7.31 (1H, t, J = 1.8 Hz), 7.24 (1H, t, J = 8.0 Hz), 7.14 (1H, d, J = 8.0 Hz), 6.57 (2H, br s), 4.12 (1H, d, J = 8.0 Hz), 2.95 (1H, dd, J = 16.2, 8.0 Hz); ¹³C NMR (100 MHz, DMSO-d6): δ 170.81, 161.40, 156.74, 155.18, 146.63, 130.77, 129.38, 129.30, 125.57, 121.78, 91.03, 38.25, 32.71.

### 2-Amino-5-(3-trifluoromethoxyphenyl)-5,8-dihydro-3H,6H-pyrido[2,3-d]pyrimidine-4,7-dione (2o):

Yield: 23%; mp: >300 °C; ¹H NMR (400 MHz, DMSO-d6): δ 10.64 (1H, br s), 10.15 (1H, s), 7.40 (1H, t, J = 8.0 Hz), 7.17 (2H, t, J = 8.0 Hz), 7.12 (1H, s), 6.58 (2H, br s), 4.18 (1H, d, J = 7.2 Hz), 2.97 (1H, dd, J = 16.2, 7.2 Hz); ¹³C NMR (100 MHz, DMSO-d6): δ 171.26, 161.71, 156.92, 155.40, 148.72, 146.70, 130.66, 120.22 (q, J = 254.60 Hz), 119.21, 119.08, 116.41, 91.23, 38.31, 32.81.

### 2-Amino-5-(3-methoxyphenyl)-5,8-dihydro-3H,6H-pyrido[2,3-d]pyrimidine-4,7-dione (2p)

Yield: 35%; mp: >300 °C; ¹H NMR (400 MHz, DMSO-d6): δ 10.60 (1H, br s), 10.07 (1H, s), 7.17 (1H, t, J = 7.6 Hz), 6.75 (1H, dd, J = 7.6, 2.4 Hz), 6.71 (1H, d, J = 2.4 Hz), 6.70 (1H, s), 6.54 (2H, br s), 4.08 (1H, d, J = 7.2 Hz), 3.69 (3H, s), 2.92 (1H, dd, J = 16.0, 7.2 Hz); ¹³C NMR (100 MHz, DMSO-d6): δ 171.24, 161.45, 159.34, 156.54, 155.11, 145.32, 129.53, 118.56, 112.85, 111.29, 91.65, 54.91, 38.51, 32.89.

### 2-Amino-5-(3-propoxyphenyl)-5,8-dihydro-3H,6H-pyrido[2,3-d]pyrimidine-4,7-dione (2q)

Yield: 32%; mp: >300 °C; ¹H NMR (400 MHz, DMSO-d6): δ 10.58 (1H, br s), 10.05 (1H, s), 7.15 (1H, t, J = 8.0 Hz), 6.74-6.67 (3H, m), 6.52 (2H, br s), 4.07 (1H, d, J = 8.0 Hz), 3.84 (2H, t, J = 6.4 Hz), 2.91 (1H, dd, J = 16.4, 8.0 Hz), 1.69 (2H, sext, J = 6.4 Hz), 0.94 (3H, t, J = 6.4 Hz); ¹³C NMR (100 MHz, DMSO-d6): δ 171.09, 161.47, 158.76, 156.62, 155.05, 145.34, 129.50, 118.54, 112.19, 111.74, 91.68, 68.76, 38.52, 32.89, 22.05, 10.47.

### 2-Amino-5-biphenyl-3-yl-5,8-dihydro-3H,6H-pyrido[2,3-d]pyrimidine-4,7-dione (2r)

Yield: 37%; mp: >300 °C; ¹H NMR (400 MHz, DMSO-d6): δ 10.58 (1H, br s), 10.09 (1H, s), 7.53 (2H, dd, J = 8.0, 1.2 Hz), 7.42 (5H, m), 7.32 (2H, t, J = 8.0 Hz), 7.01 (1H, d, J = 8.0 Hz), 6.51 (2H, brs), 4.17 (1H, d, J = 7.6 Hz), 2.95 (1H, dd, J = 16.0, 7.6 Hz); ¹³C NMR (100 MHz, DMSO-d6): δ 171.38, 161.86, 155.22, 144.66, 140.62, 140.46, 129.37, 129.21, 127.71, 126.94, 126.85, 125.65, 125.20, 91.95, 62.93, 38.71, 33.17.

### 2-Amino-5-(3-ethylphenyl)-5,8-dihydro-3H,6H-pyrido[2,3-d]pyrimidine-4,7-dione (2s)

Yield: 37% ; mp: >300 °C; ¹H NMR (400 MHz, DMSO-d6): δ 10.60 (1H, br s), 10.07 (1H, s), 7.15 (1H, t, J = 8.0 Hz), 7.01 (1H, d, J = 8.0 Hz), 7.00 (1H, s), 6.91 (1H, d, J = 8.0 Hz), 6.53 (2H, br s), 4.08 (1H, d, J = 7.2 Hz), 2.93 (1H, dd, J = 16.6, 7.2 Hz), 2.52 (2H, q, J = 7.6 Hz), 1.13 (3H, t, J = 7.6 Hz); ¹³C NMR (100 MHz, DMSO-d6): δ 171.27, 161.52, 156.60, 155.09, 143.76, 128.43, 126.14, 125.87, 123.66, 91.75, 62.82, 32.95, 28.24, 15.61.

### 2-Amino-5-(3-propylphenyl)-5,8-dihydro-3H,6H-pyrido[2,3-d]pyrimidine-4,7-dione (2t)

Yield: 18%; mp: >300 °C; ¹H NMR (400 MHz, DMSO-d6): δ 10.57 (1H, br s), 10.05 (1H, s), 7.14 (1H, t, J = 7.6 Hz), 6.98 (1H, d, J = 7.6 Hz), 6.97 (1H, s), 6.91 (1H, d, J = 7.6 Hz), 6.52 (2H, br s), 4.07 (1H, d, J = 8.0 Hz), 2.92 (1H, dd, J = 16.2, 8.0 Hz), 1.52 (2H, sext, J = 7.2 Hz), 0.86 (3H, t, J = 7.2 Hz); ¹³C NMR (100 MHz, DMSO-d6): δ 171.20, 161.48, 156.59, 155.04, 143.66, 142.24, 128.32, 126.63, 126.43, 123.73, 91.75, 38.87, 37.38, 32.90, 24.18, 13.74.

### 2-Amino-5-(3-butylphenyl)-5,8-dihydro-3H,6H-pyrido[2,3-d]pyrimidine-4,7-dione (2u)

Yield: 30%; mp: >300 °C; ¹H NMR (400 MHz, DMSO-d6): δ 10.57 (1H, br s), 10.01 (1H, s), 7.13 (1H, t, J = 7.6 Hz), 6.98 (1H, d, J = 7.6 Hz), 6.97 (1H, s), 6.90 (1H, d, J = 7.6 Hz), 6.52 (2H, br s), 4.07 (1H, d, J = 8.0 Hz), 2.92 (1H, dd, J = 16.4, 8.0 Hz), 1.48 (2H, quin, J = 7.4 Hz), 1.27 (2H, sext, J = 7.4 Hz), 0.87 (3H, t, J = 7.4 Hz); ¹³C NMR (100 MHz, DMSO-d6): δ 171.16, 161.46, 156.59, 155.04, 143.66, 142.42, 128.33, 126.59, 126.39, 123.67, 91.76, 38.70, 34.93, 33.20, 32.90, 21.82, 13.82.
1) Tu, S. et al. Bioorg. Med. Chem. Lett. 2006, 16, 3578-3581.
2) Muskinja, J. et al. Med. Chem. Res. 2016, 25, 1744-1753.
3) McDonald, B. et al. Org. Lett. 2015,17, 98-101.
4) US 2015/0210682A
5) JP 2010-526138 A
6) Wang, B. et al. Eur. J. Org. Chem. 2009, 22, 3688-3692.
7) WO 2008/136756
8) Shi, D. Q. et al. J. Heterocyclic Chem. 2009, 46, 1331-1334.

### [Example 2] Synthesis of PA-9 derivatives

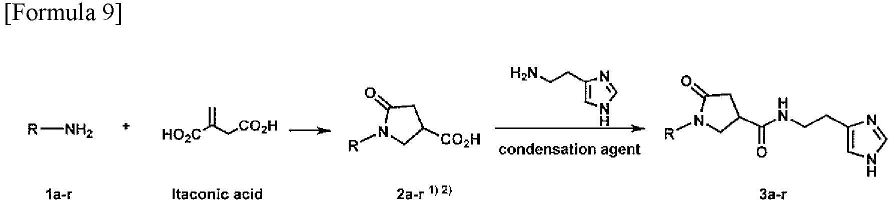

| | | |
|---|---|---|
| 1a: R=4-Cl-indazol³⁾ | 2a: R=4-Cl-indazol | 3a: R=4-Cl-indazol (--%) |
| 1b: R=S-Cl-indazol³⁾ | 2b: R=5-Cl-indazol | 3b: R=5-Cl-indazol (24% over two steps) |
| 1c: R=6-Cl-indazol³⁾ | 2c: R=6-Cl-indazol | 3c: R=6-Cl-indazol (17% over two steps) |
| 1d: R=7-Cl-indazol³⁾ | 2d: R=7-Cl-indazol | 3d: R=7-Cl-indazol (36% over two steps) |
| 1e: R=Ph | 2e: R=Ph | 3e: R=Ph (63%) |
| 1f: R=4-Me-Ph | 2f: R=4-Me-Ph | 3f: R=4-Me-Ph (93%) |
| 1g: R=4-Cl-Ph | 2g: R=4-Cl-Ph | 3g: R=4-Cl-Ph (93%) ⁵⁾ |
| 1h: R=4-F-Ph | 2h: R=4-F-Ph | 3h: R=4-F-Ph (94%) |
| 1i: R=4-OMe-Ph | 2i: R=4-OMe-Ph | 3i: R=4-OMe-Ph (95%) |
| 1j: R=4-CN-Ph | 2j: R=4-CN-Ph | 3j: R=4-CN-Ph (69%) |
| 1k: R=2-OH-Ph | 2k: R=2-OH-Ph | 3k: R=2-OH-Ph (40%) |
| 1l: R=3-OH-Ph | 2l: R=3-OH-Ph | 3l: R=3-OH-Ph (58%) |
| 1m: R=4-OH-Ph | 2m: R=4-OH-Ph | 3m: R=4-OH-Ph (54%) |
| 1n: R=pyrazol | 2n: R=pyrazol | 3n: R=pyrazol (30%) |
| 1o: R=Bn | 2o: R=Bn | 3o: R=Bn (76%) ⁶⁾ |
| 1p: R-2-OH-Bn⁴⁾ | 2p: R=2-OH-Bn | 3p: R=2-OH-Bn (30%) |
| 1q: R=3-OH-Bn⁴⁾ | 2q: R=3-OH-Bn | 3q: R=3-OH-Bn (40%) |
| 1r: R=4-OH-Bn | 2r: R=4-OH-Bn | 3r: R=4-OH-Bn (29%) |

The corresponding amine from the amines 1a to d (1.0 eq) and itaconic acid (1.0 eq) were mixed at room temperature, and the mixture was heated gradually from 60°C to 150°C in an Ar atmosphere in accordance with Literature ¹⁾ (JP 2006-510596 A). The mixture was heated at 150°C for 30 minutes and then cooled to room temperature to obtain carboxylic acids 2a to d, respectively, as pale yellow solids. To a solution of the corresponding carboxylic acid from the carboxylic acids 2a to d (1.0 eq) in DMF were sequentially added at room temperature dicyclohexylcarbodiimide (DCC) (1.2 eq), 1-hydroxybenzotriazole (HOBt) (1.2 eq) and histamine (1.2 eq), and the mixture was stirred for 15 hours. A crude product was obtained by distilling off the solvent, and the crude product was purified by silica gel chromatography (CH₂Cl₂:MeOH = 5:1) and further washed with EtOAc:MeOH = 5:1 (0.5 mL × 3) to obtain 3a to d, respectively, as white solids.

### 1-(4-chloro-1H-indazol-3-yl)-5-oxo-3-pyrrolidinecarboxylic acid (2a)

¹H NMR (400MHz, Pyridine-d5): δ 10.74 (1H, br s), 7.73 (1H, d, J = 2.1 Hz), 7.45 (1H, d, J = 8.6 Hz), 7.15 (1H, dd, J = 2.1, 8.6 Hz), 4.65 (1H, dd, J = 13.0, 7.6 Hz), 4.44 (1H, t, J = 7.6 Hz), 3.40 (1H, quint, J = 7.6 Hz), 2.77 (1H, dd, J = 17.6, 7.6 Hz), 2.60 (1H, dd, J = 17.6, 7.6 Hz).

### 1-(5-chloro-1H-indazol-3-yl)-5-oxo-3-pyrrolidinecarboxylic acid (2b)

¹H NMR (400MHz, Pyridine-d5): δ 11.53 (1H, br s), 7.38 (1H, d, J = 8.4 Hz), 7.14 (1H, dd, J = 8.4, 7.2 Hz), 6.93 (1H, d, J = 7.2 Hz), 4.67 (1H, dd, J = 13.0, 7.5 Hz), 4.52 (1H, t, J = 7.5 Hz), 3.39 (1H, quint, J = 7.5 Hz), 2.76 (1H, dd, J = 17.5, 7.5 Hz), 2.57 (1H, dd, J = 17.5, 7.5 Hz).

### 1-(6-chloro-1H-indazol-3-yl)-5-oxo-3-pyrrolidinecarboxylic acid (2c)

¹H NMR (400MHz, Pyridine-d5): δ 11.63 (1H, br s), 7.71 (1H, d, J = 9.0 Hz), 7.48 (1H, d, J = 1.7 Hz), 6.88 (1H, dd, J = 9.0, 1.7 Hz), 4.65 (1H, dd, J = 13.0, 7.7 Hz), 4.44 (1H, t, J = 7.7 Hz), 3.40 (1H, quint, J = 7.7 Hz), 2.68 (1H, dd, J = 17.1, 7.7 Hz), 2.60 (1H, dd, J = 17.1, 7.7 Hz).

### 1-(7-chloro-1H-indazol-3-yl)-5-oxo-3-pyrrolidinecarboxylic acid (2d)

¹H NMR (400MHz, Pyridine-d5): δ 11.66 (1H, br s), 7.67 (1H, d, J = 8.7 Hz), 7.30 (1H, d, J = 7.3 Hz), 6.86 (1H, dd, J = 8.7, 7.3 Hz), 4.71 (1H, dd, J = 13.4, 7.4 Hz), 4.48 (1H, t, J = 7.4 Hz), 3.42 (1H, quint, J = 7.4 Hz), 2.78 (1H, dd, J = 17.6, 7.4 Hz), 2.61 (1H, dd, J = 17.6, 7.4 Hz).

### N-[2-(1H-imidazol-4-yl)ethyl]-1-(4-chloro-1H-indazol-3-yl)-5-oxo-3-pyrrolidinecarboxamide (3a)

mp: 190-191 °C; IR (KBr): 3566, 3437, 3306, 1695, 1636, 1558, 1508 cm⁻¹; ¹H NMR (400MHz, Pyridine-d5): δ 9.00 (1H, br s), 7.96 (1H, s), 7.61 (1H, d, J = 7.4 Hz), 7.14 (1H, t, J = 7.4 Hz), 7.15(1H, s), 6.98 (1H, d, J = 7.4 Hz), 4.72 (1H, t, J = 9.1 Hz), 3.96-3.85 (3H, m), 3.26 (1H, dd, J = 15.6, 9.1 Hz), 3.11 (2H, t, J = 6.8 Hz), 2.78 (1H, dd, J = 15.6, 9.1 Hz); ¹³C NMR (100 MHz, DMSO-d6): δ 169.56, 168.62, 147.59, 134.59, 131.61, 126.70, 123.80, 119.28, 116.97, 115.67, 107.11, 48.23, 36.47, 32.61, 26.57.

### N-[2-(1H-imidazol-4-yl)ethyl]-1-(5-chloro-1H-indazol-3-yl)-5-oxo-3-pyrrolidinecarboxamide (3b)

Yield: 24% over two steps; mp: 208-209 °C; IR (KBr): 3735, 3649, 3097, 1684, 1653, 1558, 1508 cm⁻¹; ¹H NMR (400MHz, Pyridine-d5): δ 9.04 (1H, br s), 8.05 (1H, s), 7.69 (1H, d, J = 9.6 Hz), 7.68 (1H, s), 7.27 (1H, d, J = 9.6 Hz), 7.71(1H, s), 5.09 (1H, dd, J = 13.6, 8.4 Hz), 4.73 (1H, t, J = 8.4 Hz), 3.94-3.86 (3H, m), 3.28 (1H, dd, J = 14.8, 8.4 Hz), 3.11 (2H, t, J = 6.6 Hz), 2.84 (1H, dd, J = 14.8, 8.4 Hz); ¹³C NMR (100 MHz, DMSO-d6): δ 169.63, 168.21, 145.55, 134.50, 133.79, 131.92, 127.15, 123.19, 118.50, 118.39, 116.85, 108.13, 48.33, 38.747, 36.33, 32.92, 26.53.

### N-[2-(1H-imidazol-4-yl)ethyl]-1-(6-chloro-1H-indazol-3-yl)-5-oxo-3-pyrrolidinecarboxamide (3c)

Yield: 17% over two steps; mp: 196-198 °C; IR(KBr): 3290, 3213, 3101, 1683, 1636, 1558, 1508 cm⁻¹; ¹H NMR (400MHz, Pyridine-d5): δ 9.12 (1H, br s), 8.30 (1H, s), 7.83 (1H, s), 7.66 (1H, d, J = 9.2 Hz), 7.25 (1H, s), 6.96 (1H, d, J = 9.2 Hz), 5.03 (1H, dd, J = 13.0, 8.3 Hz), 4.71 (1H, t, J = 8.3 Hz), 3.91-3.84 (3H, m), 3.27 (1H, dd, J = 15.2, 8.3 Hz), 3.12 (2H, t, J = 6.4 Hz), 2.87 (1H, dd, J = 15.2, 8.3 Hz); ¹³C NMR (100 MHz, DMSO-d6): δ 169.79, 168.28, 147.25, 134.03, 132.74, 132.42, 131.35, 121.77, 119.93, 116.53, 115.03, 106.65, 48.23, 38.21, 36.25, 32.92, 25.47.

### N-[2-(1H-imidazol-4-yl)ethyl]-1-(7-chloro-1H-indazol-3-yl)-5-oxo-3-pyrrolidinecarboxamide (3d)

Yield: 36% over two steps; mp: 238-239 °C; IR(KBr): 3319, 3231, 3213, 1663, 1636, 1558, 1508 cm⁻¹; ¹H NMR (400MHz, Pyridine-d5): δ 9.02 (1H, br s), 8.49 (1H, s), 7.76 (1H, s), 7.52 (1H, d, J = 7.5 Hz), 7.40 (1H, d, J = 7.5 Hz), 6.98 (1H, s), 6.88 (1H, t, J = 7.5 Hz), 5.13 (1H, dd, J = 14.6, 8.5 Hz), 4.81 (1H, t, J = 8.5 Hz), 3.95-3.86 (3H, m), 3.26 (1H, dd, J = 16.2, 8.5 Hz), 3.11 (2H, t, J = 6.8 Hz), 2.83 (1H, dd, J = 16.2, 8.5 Hz); ¹³C NMR (100 MHz, DMSO-d6): δ 169.56, 168.32, 144.32, 134.51, 133.83, 133.30, 125.80, 120.48, 119.42, 119.04, 116.84, 109.42, 48.36, 38.77, 36.32, 32.95, 26.57.

To an aqueous solution of the corresponding amine from the amines 1e to j (1.0 eq) (3 mL) was added at room temperature itaconic acid (1.2 eq), and the mixture was heated and refluxed with stirring for 20 hours in an Ar atmosphere in accordance with Literature ²⁾ (JP 2012-529476 A). The mixture was cooled to room temperature, and a depositing solid was then filtered to obtain carboxylic acids 2e to j, respectively. To a solution of the corresponding carboxylic acid from the carboxylic acids 2e to j (1.0 eq) in a mixture of CH₂Cl₂ and DMF were sequentially added at room temperature 1-ethyl-3-(3-dimethylaminopropyl)carbodiimide (EDC) (1.2 eq), 4-dimethylaminopyridine (DMAP) (0.1 eq) and histamine (1.2 eq), and the mixture was stirred for 15 hours. A crude product was obtained by distilling off the solvent, and purified by silica gel chromatography (CH₂Cl₂:MeOH = 10:1) to obtain 3e to j, respectively, as white solids.

### N-[2-(1H-imidazol-4-yl)ethyl]-1-phenyl-5-oxo-3-pyrrolidinecarboxamide (3e)

Yield: 63%; mp: 149-151 °C; IR (KBr): 3675, 3306, 1678, 1643, 1558 cm⁻¹; ¹H NMR (400MHz, Pyridine-d5): δ 9.11 (1H, t, J = 5.8 Hz),7.90 (1H, s), 7.72 (2H, d, J = 8.4 Hz), 7.28 (2H, t, J = 8.4 Hz), 7.09 (1H, s), 7.06 (1H, t, J = 8.4 Hz), 4.13 (1H, dd, J = 9.6, 8.3 Hz), 3.93 (1H, t, J = 8.3 Hz), 3.83 (2H, q, J = 7.5 Hz), 3.47 (1H, quint, J = 8.3 Hz), 3.14 (1H, dd, J = 16.7, 8.3 Hz), 3.08 (2H, t, J = 7.5 Hz), 2.84 (1H, dd, J = 16.7, 8.3 Hz); ¹³C NMR (100 MHz, DMSO-d6): δ 172.16, 171.96, 139.22, 134.58, 133.95, 128.70, 124.00, 119.36, 116.83, 50.73, 38.66, 35.66, 35.43, 26.38.

### N-[2-(1H-imidazol-4-yl)ethyl]-1-(4-methylphenyl)-5-oxo-3-pyrrolidinecarboxamide (3f)

Yield: 93%; mp: 176-178 °C; IR (KBr): 3306, 3088, 1675, 1639, 1556 cm⁻¹; ¹H NMR (400 MHz, Pyridine-d5): δ 9.10 (1H, t, J = 5.8 Hz), 7.91 (1H, s), 7.64 (2H, d, J = 7.2 Hz), 7.08 (2H, t, J = 7.2 Hz), 7.07 (1H, s), 4.14 (1H, dd, J = 9.6, 8.0 Hz), 3.92 (1H, t, J = 8.0 Hz), 3.83 (2H, q, J = 7.2 Hz), 3.46 (1H, quint, J = 8.0 Hz), 3.14 (1H, dd, J = 17.6, 8.0 Hz), 3.08 (2H, t, J = 7.2 Hz), 2.84 (1H, dd, J = 17.6, 8.0 Hz), 2.13 (3H, s); ¹³C NMR (100 MHz, DMSO-d6): δ 172.17, 172.10, 137.00, 134.86, 134.44, 133.26, 129.30, 119.58, 117.04, 51.00, 39.17, 36.02, 35.84, 27.01, 20.63.

### 1-(4-chlorophenyl)-N-[2-(1H-imidazol-4yl)ethyl]- 5-oxo-3-pyrrolidinecarboxamide (3g)

Yield: 93%; mp: 196-198 °C; IR (KBr): 3119, 3017, 1695, 1647, 1558 cm⁻¹; ¹H NMR (400MHz, Pyridine-d5): δ 9.16 (1H, t, J = 5.2 Hz), 7.95 (1H, s), 7.71 (2H, d, J = 8.4 Hz), 7.30 (2H, d, J = 8.4 Hz), 7.11 (1H, s), 4.10 (1H, dd, J = 9.4, 8.3 Hz), 3.92 (1H, t, J = 8.3 Hz), 3.83 (2H, q, J = 6.7 Hz), 3.50 (1H, quint, J = 8.3 Hz), 3.11 (1H, dd, J = 17.5, 8.3 Hz), 3.09 (2H, t, J = 5.7 Hz), 2.85 (1H, dd, J = 17.5, 8.3 Hz); ¹³C NMR (100 MHz, DMSO-d6): δ 172.20, 171.68, 137.90, 134.43, 133.95, 128.36, 127.49, 120.60, 116.62, 50.47, 38.74, 35.62, 35.30, 26.52.

### N-[2-(1H-imidazol-4-yl)ethyl]-1-(4-fluorophenyl)-5-oxo-3-pyrrolidinecarboxamide (3h)

Yield: 94%; mp: 232-234 °C; IR (KBr): 3140, 3126, 1688, 1645, 1570 cm⁻¹; ¹H NMR (400MHz, Pyridine-d5): δ 9.13 (1H, t, J = 4.8 Hz), 7.72-7.68 (2H, m), 7.92 (1H, s), 7.11 (1H, s), 7.09-7.04 (2H, m), 4.12 (1H, dd, J = 12.2, 8.7 Hz), 3.92 (1H, t, J = 8.7 Hz), 3.84 (2H, t, J = 7.0 Hz), 3.49 (1H, quint, J = 8.7 Hz), 3.14 (1H, dd, J = 17.2, 8.7 Hz), 3.09 (2H, t, J = 7.0 Hz), 2.85 (1H, dd, J = 17.2, 8.7 Hz); ¹³C NMR (100 MHz, DMSO-d6): δ 172.05, 171.90, 158.44 (d, J = 240.3 Hz), 135.64, 134.62, 134.14, 121.35 (d, J = 7.6 Hz), 116.81, 115.25 (d, J = 22.0 Hz), 50.92, 38.92, 35.68, 35.58, 26.72.

### N-[2-(1H-imidazol-4-yl)ethyl]-1-(4-methoxyphenyl)-5-oxo-3pyrrolidinecarboxamide (3i)

Yield: 95%; mp: 151-153 °C; IR (KBr): 3239, 3075, 1684, 1635, 1568 cm⁻¹; ¹H NMR (400 MHz, Pyridine-d5): δ 9.08 (1H, t, J = 5.2 Hz), 7.91 (1H, s), 7.68 (2H, d, J = 8.6 Hz), 7.10 (1H, s), 6.92 (2H, d, J = 8.6 Hz),4.16 (1H, dd, J = 9.4, 8.4 Hz), 3.93 (1H, t, J = 8.4 Hz), 3.84 (2H, q, J = 7.6 Hz), 3.62 (3H, s), 3.46 (1H, quint, J = 8.4 Hz), 3.15 (1H, dd, J = 17.1, 8.4 Hz), 3.09 (2H, t, J = 7.6 Hz), 2.84 (1H, dd, J = 17.1, 8.4 Hz); ¹³C NMR (100 MHz, DMSO-d6): δ 171.97, 171.57, 155.80, 134.65, 134.26, 132.41, 121.20, 116.80, 113.82, 55.20, 51.03, 38.95, 35.68, 35.62, 26.81.

### N-[2-(1H-imidazol-4-yl)ethyl]-1-(4-cyanophenyl)-5-oxo-3-pyrrolidinecarboxamide (3j)

Yield: 69%; mp: 211-212 °C; IR (KBr): 3151, 3019, 2231, 1703, 1646, 1558 cm⁻¹; ¹H NMR (400MHz, Pyridine-d5): δ 9.17 (1H, t, J = 5.2 Hz), 7.92 (1H, s), 7.83 (2H, d, J = 8.8 Hz), 7.60 (2H, d, J = 8.8 Hz), 7.11 (1H, s), 4.12 (1H, dd, J = 9.6, 8.4 Hz), 3.96 (1H, t, J = 8.4 Hz), 3.84 (2H, t, J = 7.0 Hz), 3.51 (1H, quint, J = 8.4 Hz), 3.16 (1H, dd, J = 17.2, 8.4 Hz), 3.10 (2H, t, J = 7.0 Hz), 2.88 (1H, dd, J = 17.2, 8.4 Hz); ¹³C NMR (100 MHz, DMSO-d6): δ 173.28, 171.75, 143.03, 134.67, 133.06, 119.03, 118.93, 105.53, 51.51, 38.98, 36.03, 35.39, 26.85.

The corresponding amine from the amines 1k to m (1.0 eq) and itaconic acid (1.0 eq) were mixed at room temperature, and the mixture was heated gradually from 60°C to 150°C in an Ar atmosphere in accordance with Literature ¹⁾ (JP 2006-510596 A). The mixture was heated at 150°C for 30 minutes and then cooled to room temperature to obtain carboxylic acids 2k to m, respectively. To a solution of the corresponding carboxylic acid from the carboxylic acids 2k to m (1 eq) in a mixture of CH₂Cl₂ and DMF were sequentially added at room temperature EDC (1.2 eq), DMAP (0.1 eq) and histamine (1.2 eq), and the mixture was stirred for 15 hours. A crude product was obtained by distilling off the solvent, and purified by silica gel chromatography (CH₂Cl₂:MeOH = 10:1) to obtain 3k to m as white, yellow and red foamy solids, respectively.

### N-[2-(1H-imidazol-4-yl)ethyl]-1-(2-hydroxyphenyl)-5-oxo-3-pyrrolidinecarboxamide (3k)

Yield: 40%; IR (KBr): 3651, 3265, 3213, 1684, 1670, 1558 cm⁻¹; ¹H NMR (400 MHz, Pyridine-d5): δ 9.08 (1H, br s), 7.91 (1H, s), 7.42 (1H, d, J = 8.8 Hz), 7.21-7.15 (2H, m), 7.10 (1H, s), 6.92-6.88 (1H, m), 4.30 (1H, dd, J = 9.2, 7.5 Hz), 4.10 (1H, t, J = 7.5 Hz), 3.85 (2H, q, J = 6.4 Hz), 3.47 (1H, quint, J = 7.5 Hz), 3.13 (1H, dd, J = 16.4, 7.5 Hz), 3.06 (2H, t, J = 6.4 Hz), 2.83 (1H, dd, J = 16.4, 7.5 Hz); ¹³C NMR (100 MHz, DMSO-d6): δ 172.52, 172.22, 152.77, 134.68, 128.30, 128.24, 125.48, 119.12, 118.74, 116.87, 116.74, 51.68, 38.98, 37.09, 34.39, 26.85.

### N-[2-(1H-imidazol-4-yl)ethyl]-1-(3-hydroxyphenyl)-5-oxo-3-pyrrolidinecarboxamide (3l)

Yield: 58%; IR (KBr): 3790, 3439, 3337, 1684, 1653, 1558 cm⁻¹; ¹H NMR (400 MHz, Pyridine-d5): δ 8.97 (1H, br s), 8.09 (1H, s), 7.93 (1H, s), 7.28 (1H, t, J = 8.2 Hz), 7.20 (1H, d, J = 8.2 Hz), 7.13 (1H, s), 6.98 (1H, d, J = 8.2 Hz), 4.29 (1H, dd, J = 9.0, 8.3 Hz), 3.98 (1H, t, J = 8.3 Hz), 3.89 (2H, q, J = 6.9 Hz), 3.36 (1H, quint, J = 8.3 Hz), 3.21 (1H, dd, J = 17.1, 8.3 Hz), 3.09 (2H, t, J = 6.9 Hz), 2.82 (1H, dd, J = 17.1, 8.3 Hz); ¹³C NMR (100 MHz, DMSO-d6): δ 172.07, 171.93, 157.55, 140.28, 134.68, 129.38, 111.17, 109.78, 106.63, 50.84, 39.00, 35.99, 35.54, 26.89.

### N-[2-(1H-imidazol-4-yl)ethyl]-1-(4-hydroxyphenyl)-5-oxo-3-pyrrolidinecarboxamide (3m)

Yield: 54%; IR (KBr): 3585, 3251, 3190, 1684, 1653, 1558 cm⁻¹; ¹H NMR (400 MHz, Pyridine-d5): δ 11.49 (1H, br s), 8.97 (1H, br s), 7.93 (1H, s), 7.76 (2H, dd, J = 8.6, 2.4 Hz), 7.15 (2H, dd, J = 8.6, 2.4 Hz), 7.14 (1H, s), 4.29 (1H, td, J = 8.4, 2.1 Hz), 3.95 (1H, td, J = 8.4, 2.1 Hz), 3.90 (2H, q, J = 6.3 Hz), 3.39 (1H, quint, J = 8.4 Hz), 3.22 (1H, ddd, J = 16.7, 8.4, 2.1 Hz), 3.10 (2H, t, J = 6.3 Hz), 2.84 (1H, ddd, J = 16.7, 8.4, 2.1 Hz); ¹³C NMR (100 MHz, DMSO-d6): δ 172.52, 171.86, 154.61, 135.20, 131.48, 122.05, 115.61, 51.67, 39.49, 36.23, 36.09, 27.40.

An amine 1n (1.0 eq) and itaconic acid (1.0 eq) were mixed at room temperature, and the mixture was heated gradually from 60°C to 150°C in an Ar atmosphere in accordance with Literature ¹⁾ (JP 2006-510596 A). The mixture was heated at 150°C for 30 minutes and then cooled to room temperature to obtain a carboxylic acid 2n. To a solution of the carboxylic acid 2n (1.0 eq) in a mixture of CH₂Cl₂ and DMF were sequentially added at room temperature DCC (1.2 eq), HOBt (1.2 eq) and histamine (1.2 eq), and the mixture was stirred for 15 hours. A crude product was obtained by distilling off, and purified by silica gel chromatography (CH₂Cl₂:MeOH = 10:1) to obtain 3n as a white solid.

### N-[2-(1H-imidazol-4-yl)ethyl]-1-(1H-pyrazol-3-yl)-5-oxo-3-pyrrolidinecarboxamide (3n)

Yield: 30%; mp: 213-211 °C; IR (KBr): 3676, 3320, 3203, 1689, 1652, 1635, 1557 cm⁻¹; ¹H NMR (400 MHz, Pyridine-d5): δ 12.24 (1H, br s), 9.00 (1H, br s), 8.03 (1H, s), 7.88 (1H, s), 7.56 (1H,s), 7.10(1H, s), 4.78 (1H, dd, J = 12.6, 8.1 Hz), 4.26 (1H, t, J = 8.1 Hz), 3.82 (2H, q, J = 6.7 Hz), 3.69 (1H, quint, J = 8.1 Hz), 3.22 (1H, dd, J = 15.3, 8.1 Hz), 3.06 (2H, t, J = 6.7 Hz), 2.72 (1H, dd, J = 15.3, 8.1 Hz); ¹³C NMR (100 MHz, DMSO-d6): δ 169.67, 168.26, 162.33, 138.77, 138.73, 134.64, 134.53, 89.01, 47.04, 38.87, 36.72, 33.00, 26.97.

The corresponding amine from the amines 1o to r (1.0 eq) and itaconic acid (1.0 eq) were mixed at room temperature, and the mixture was heated gradually from 60°C to 150°C in an Ar atmosphere in accordance with Literature ¹⁾ (JP 2006-510596 A). The mixture was heated at 150°C for 30 minutes, then cooled to room temperature and purified by silica gel chromatography (CH₂Cl₂:MeOH = 30:1) to obtain carboxylic acids 2o to r, respectively. To a solution of the corresponding carboxylic acid from the carboxylic acids 2o to r (1.0 eq) in a mixture of CH₂Cl₂ and DMF were sequentially added at room temperature DCC (1.2 eq), HOBt (1.2 eq) and histamine (1.2 eq), and the mixture was stirred for 15 hours. A crude product was obtained by distilling off the solvent, and purified by silica gel chromatography (CH₂Cl₂:MeOH = 10:1) to obtain 3o to r, respectively, as a yellow solid or a white foamy solid.

### N-[2-(1H-imidazol-4yl)ethyl]-5-oxo-1-(phenylmethyl)-3-pyrrolidinecarboxamide (3o)

Yield: 29%; IR (KBr): 3271, 3155, 1670, 1652, 1558 cm⁻¹; ¹H NMR (400MHz, Pyridine-d5): δ 8.85 (1H, br), 7.92 (1H, s), 7.33-7.25 (5H, m), 7.08 (1H, s), 4.56 (1H, d, J = 14.6 Hz), 4.48 (1H, d, J = 14.6 Hz), 3.85 (2H, q, J = 6.5 Hz), 3.67 (1H, t, J = 8.0 Hz), 3.39 (1H, t, J = 8.0 Hz), 3.25 (1H, quint, J = 8.0 Hz), 3.10 (1H, dd, J =17.0, 8.0 Hz), 3.05 (2H, t, J = 6.5 Hz), 2.72 (1H, dd, J = 17.0, 8.0 Hz).

### N-[2-(1H-imidazol-4-yl)ethyl]-1-[(2-hydroxyphenyl)methyl]-5-oxo-3-pyrrolidinecarboxamide (3p)

Yield: 30%; IR (KBr): 3748, 3738, 3651, 1684, 1653, 1558 cm⁻¹; ¹H NMR (400MHz, Pyridine-d5): δ 11.36 (1H, br s) 8.81 (1H, t, J = 5.8 Hz), 7.84 (1H, d, J = 1.2 Hz), 7.34 (1H, dd, J = 7.5, 1.3 Hz), 7.13 (1H, td, J = 7.5, 1.3 Hz), 7.08 (1H, dd, J = 7.5, 1.3 Hz), 7.03 (1H, s), 6.81 (1H, td, J = 1.3, 7.5 Hz), 4.74 (1H, d, J = 15.2 Hz), 4.65 (1H, d, J = 15.2 Hz), 3.82 (1H, dd, J = 9.6, 8.2 Hz), 3.77 (2H, q, J = 6.6 Hz), 3.57 (1H, t, J = 8.2 Hz), 3.22 (1H, quint, J = 8.2 Hz), 3.03 (1H, dd, J = 16.5, 8.2 Hz), 2.99 (2H, t, J = 6.6 Hz), 2.63 (1H, dd, J = 16.5, 8.2 Hz); ¹³C NMR (100 MHz, DMSO-d6): δ 172.57, 172.18, 155.26, 134,67, 128.78, 128.38, 122.58, 119.03, 115.21, 49.63, 40.57, 38.94, 35.93, 33.91, 26.87.

### N-[2-(1H-imidazol-4-yl)ethyl]-1-[(3-hydroxyphenyl)methyl]-5-oxo-3-pyrrolidinecarboxamide (3q)

Yield: 40%; IR (KBr): 3734, 3647, 3623, 1684, 1653, 1558 cm⁻¹; ¹H NMR (400MHz, Pyridine-d5): δ 8.83 (1H, br s), 7.93 (1H, s), 7.24 (2H, d, J = 7.9 Hz), 7.22 (1H, s), 7.09 (1H, s), 7.08 (1H, d, J = 7.9 Hz), 6.88 (1H, d, J = 7.9 Hz), 4.57 (1H, d, J = 14.6 Hz), 4.48 (1H, d, J = 14.6 Hz), 3.84 (2H, q, J = 6.1 Hz), 3.72 (1H, t, J = 8.1 Hz), 3.45 (1H, t, J = 8.1 Hz), 3.23 (1H, quint, J = 8.1 Hz), 3.09 (1H, dd, J = 16.0, 8.1 Hz), 3.05 (2H, t, J = 6.1 Hz), 2.67 (1H, dd, J = 16.0, 8.1 Hz); ¹³C NMR (100 MHz, DMSO-d6): δ 172.31, 172.05, 157.63, 138.14, 134.66, 134.18, 129.55, 118.17, 116.90, 114.41, 114.30, 49.14, 45.31, 38.88, 35.89, 26.78.

### N-[2-(1H-imidazol-4-yl)ethyl]-1-[(4-hydroxyphenyl)methyl]-5-oxo-3-pyrrolidinecarboxamide (3r)

Yield: 29%; IR (KBr): 3651, 3271, 3213, 1663, 1653, 1558 cm⁻¹; ¹H NMR (400MHz, Pyridine-d5): δ 8.85 (1H, br s), 7.91 (1H, s), 7.28 (2H, d, J = 7.6 Hz), 7.10 (2H, d, J = 7.6 Hz), 4.55 (1H, d, J = 14.6 Hz), 4.44 (1H, d, J = 14.6 Hz), 3.91-3.71 (2H, m), 3.70 (1H, dd, J = 8.8, 7.9 Hz), 3.45 (1H, t, J = 7.9 Hz), 3.26 (1H, quint, J = 7.9 Hz), 3.11 (1H, dd, J = 15.0, 7.9 Hz), 3.06 (2H, t, J = 7.4 Hz), 2.71 (1H, dd, J = 15.0, 7.9 Hz); ¹³C NMR (100 MHz, DMSO-d6) : δ 172.15, 156.66, 134.68, 129.07, 126.80, 115.30, 48.93, 44.83, 38.94, 35.83, 34.03, 26.89.
1) JP 2006-510596 A
2) JP 2012-529476 A
3) Saczewski, F. et al. Bioorg. Med. Chem. 2011, 19, 321-329
4) Mestichelli, P. et al. Org. Lett. 2013, 15, 5448-5451
5) Commercially available, Aurora Building Blocks, A17.818.885
6) Commercially available, Aurora Building Blocks, A21.884.126

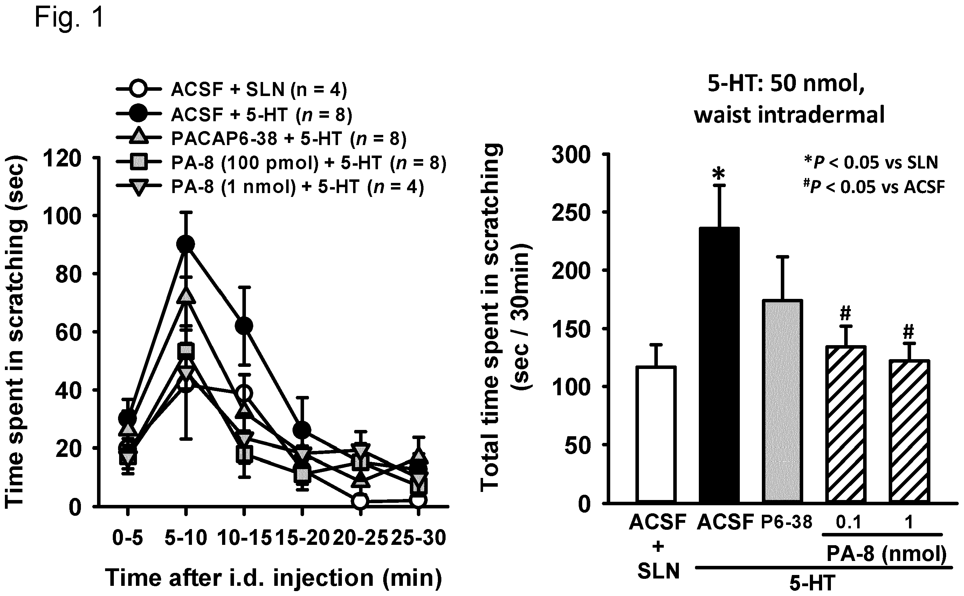

### [Example 3] Drug efficacy evaluation using the serotonin (5-HT)-induced itch mouse model (Figure 1)

5-HT (50 nmol, 10 µL) was intradermally injected into the waist of each mouse (intradermal injection, i.d.). The reaction of the mice to bite, lick or scratch the injected site was defined as an itch behavior, and the action time was measured every 5 minutes. The intrathecal pre-administration (30 minutes before the intradermal injection) of PA-8 (0.1 or 1 nmol) almost completely suppressed the 5-HT-induced itch behavior of the mice. On the other hand, the pre-administration of PACAP 6-38 (P6-38: 100 pmol) as a peptidic PACAP receptor antagonist did not provide significant effects. SLN indicates the effects obtained in the case of administering normal saline instead of 5-HT. ACSF is an abbreviation of an artificial cerebrospinal fluid, which was a solvent for P6-38 and PA-8.

### [Example 4] Drug efficacy evaluation using the dry skin itch mouse model

The present test was carried out with reference to the method of Miyamoto et al. that is a test system published as a method for evaluating itch in animals (Miyamoto T., Nojima H., Shinkado T., et al.: Itch-associated response induced by experimental dry skin in mice, Jpn. J. Phamacol., 88, 285-292, 2002).

Five-week-old ICR mice (40 mice) were each placed in a cage, and were then conditioned for 1 week. Thereafter, three days before initiation of the test, the rostral back of each mouse was shaved (treatment method A). The thus shaved 40 mice were divided into 5 groups (8 mice/group), namely, into a 3 mg/kg PA-8 oral (p.o.) administration group, a 10 mg/kg PA-8 oral (p.o.) administration group, a 30 mg/kg PA-8 oral (p.o.) administration group, a comparative group, and a control group.

### < PA-8 administration groups >

In the PA-8 administration groups, the mice, which had undergone a shaving treatment (treatment method A), were subjected to ether anesthesia. Thereafter, a cotton (2 x 2 cm) impregnated with a mixed solution of acetone (A) : diethyl ether (E) (1 : 1) was applied to the rostral back of each mouse for 15 seconds, and then, a cotton impregnated with ion exchange water (W) was applied thereto for 30 seconds [hereinafter, a series of treatments are abbreviated as an AEW treatment (treatment method B)]. The AEW treatment was carried out twice a day, with intervals of 8 hours, for 5 days. Thus, the AEW treatment was carried out a total of 10 times. On the day following the AEW treatment, PA-8 was orally administered to the mice at a dose of 3 mg/kg, 10 mg/kg, or 30 mg/kg, and from 30 minutes after the administration, the behavior of the mice was observed for 1 hour (treatment method C).

### < Comparative group >

In the comparative group, the above-described AEW treatment (treatment method B) was carried out on the mice, which had undergone a shaving treatment (treatment method A), and thereafter, a 10% DMSO aqueous solution (a solvent) was orally administered to the mice.

### < Control group >

In the control group (Naive), only the shaving treatment (treatment method A) was carried out on the mice.

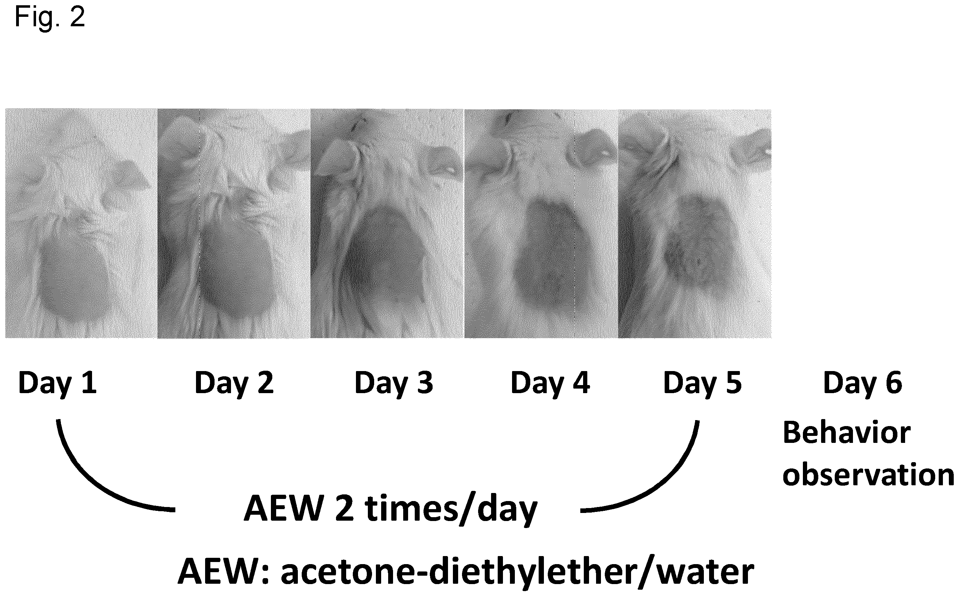

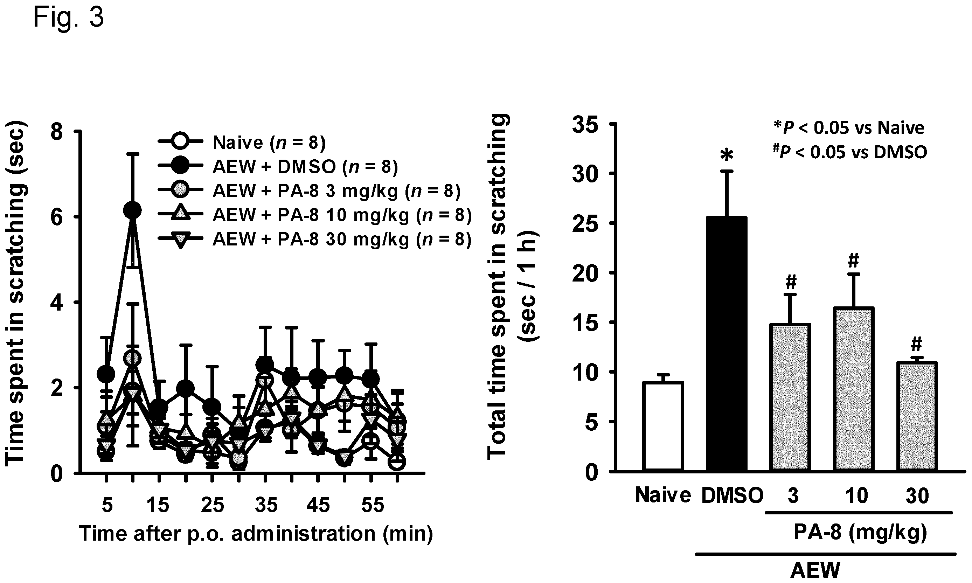

An outline of the procedures of a behavioral experiment using the dry skin itch mouse model is shown in Figure 2, and the results obtained by evaluating the time spent in scratching of each group are shown in Figure 3.

As shown in Figure 3, it is found that the scratching behavior induced by the AEW treatment was significantly suppressed by oral administration of PA-8.

### [Example 5] Drug efficacy evaluation 1 using PACAP receptor-expressing, cultured cells

The effect of each compound on the phosphorylation of CREB (cAMP-responsive element-binding protein) produced by a PACAP stimulus was examined by Western blot analysis using an anti-phosphorylated CREB (pCREB) antibody with a mouse PAC1 receptor-expressing CHO cells (PAC1/CHO cells) and a mouse VPAC1 receptor-expressing CHO cells (VPAC1/CHO cells).

Specifically, treatment with a compound (PA-8, compound 2j and compound 2o) at 10 pM to 10 nM or a solvent therefor, DMSO (VEH: 0.1% DMSO-containing phosphate buffer solution) was performed for 30 minutes, 1 nM PACAP was then added, and protein was recovered within 30 minutes after a PACAP stimulus.

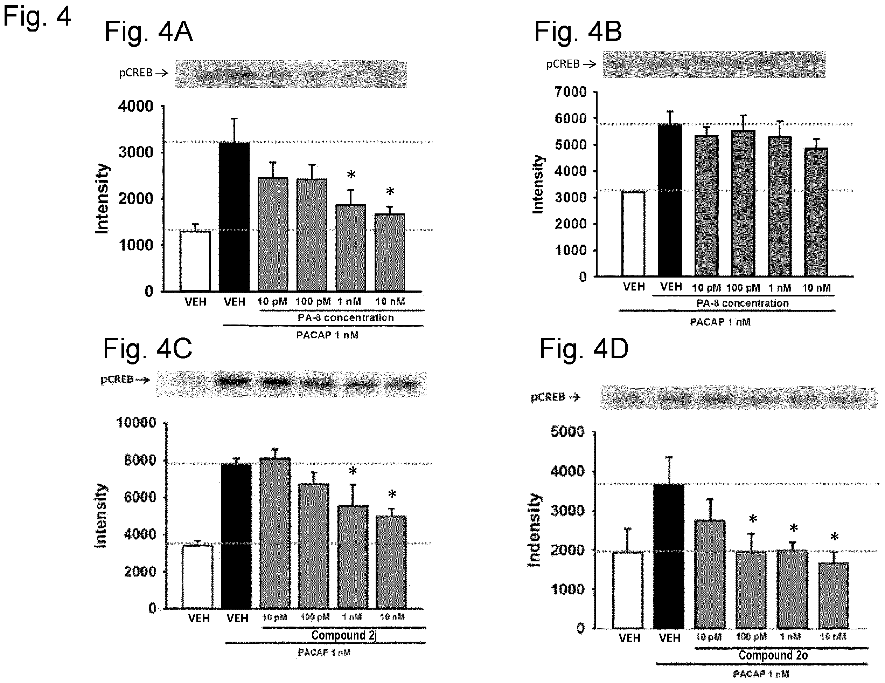

Figure 4A shows data in the PAC1/CHO cells, and PA-8 suppressed CREB phosphorylation by PACAP (1 nM) at the concentration at which PA-8 was used (10 pM to 10 nM) (n = 5; Figure 4A). Meanwhile, in the examination using the VPAC1/CHO cells, PA-8 did not suppress CREB phosphorylation by PACAP (1 nM) at the concentration at which PA-8 was used (10 pM to 10 nM) (n = 3; Figure 4B). That is, it can be said that PA-8 is a PAC1 receptor selective antagonist.

Figure 4C shows the effects of the compound 2j (10 pM to 10 nM) in the PAC1/CHO cells, and CREB phosphorylation by PACAP (1 nM) was suppressed depending on the concentration (n = 4). Figure 4D shows the effects of the compound 2o (10 pM to 10 nM), similarly. The compound 2o also suppressed CREB phosphorylation by PACAP (1 nM) at the concentration at which the compound 2o was used (10 pM to 10 nM) (n = 4). In particular, the compound 2o exhibited a slightly stronger effect of suppressing CREB phosphorylation than PA-8 did.

As PA-8 used in the above-described experiment, a commercially available compound purchased from NAMIKI SHOJI CO., LTD. was used.

### [Example 6] Drug efficacy evaluation 2 using PACAP receptor-expressing, cultured cells

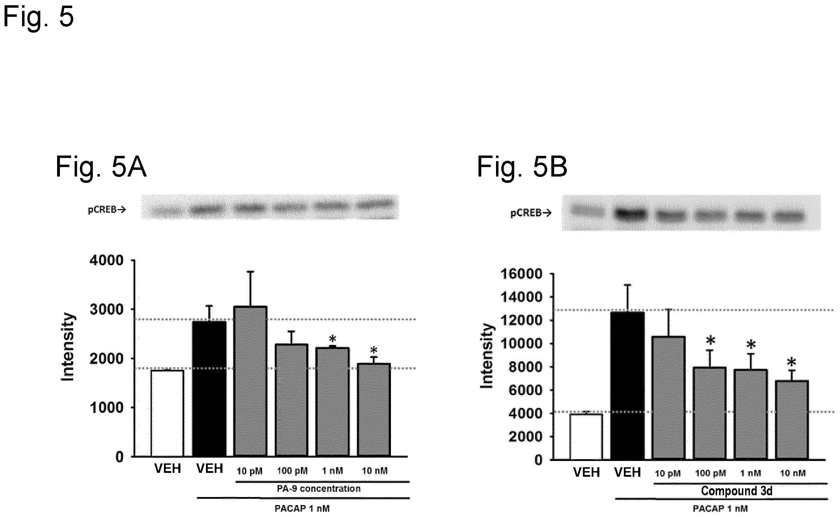

The effect of each compound on the phosphorylation of CREB (cAMP-responsive element-binding protein) produced by a PACAP stimulus was examined by Western blot analysis using an anti-phosphorylated CREB (pCREB) antibody with a mouse PAC1 receptor-expressing CHO cells (PAC1/CHO cells). Figures 5A and 5B show the effects of PA-9 and the compound 3d (10 pM to 10 nM) in the PAC1/CHO cells, respectively, and in both cases, CREB phosphorylation by PACAP (1 nM) was suppressed depending on the concentration (n = 4 to 5).

### [Example 7] Effect of PACAP waist intradermal administration

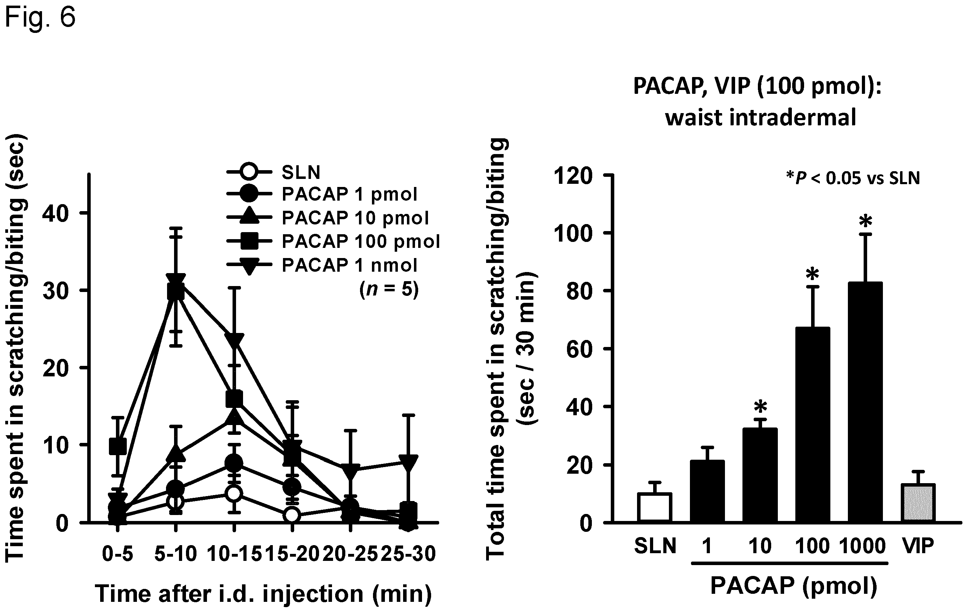

Upon waist intradermal administration (10 µL) of PACAP (1 to 1,000 pmol), the mice showed itch behavior, and they bit, licked or scratched the administered site in a dose-dependent manner. In contrast, upon waist intradermal administration of vasoactive intestinal peptide (VIP) (100 pmol), such effect was not observed. Accordingly, it is considered that this effect is mediated by the PAC1 receptors (Figure 6).

### [Example 8]

The itch behavior induced by the PACAP intradermal administration is suppressed by simultaneous intradermal administration of PA-8 with PACAP or subcutaneous pre-administration of naltrexone (NTX).

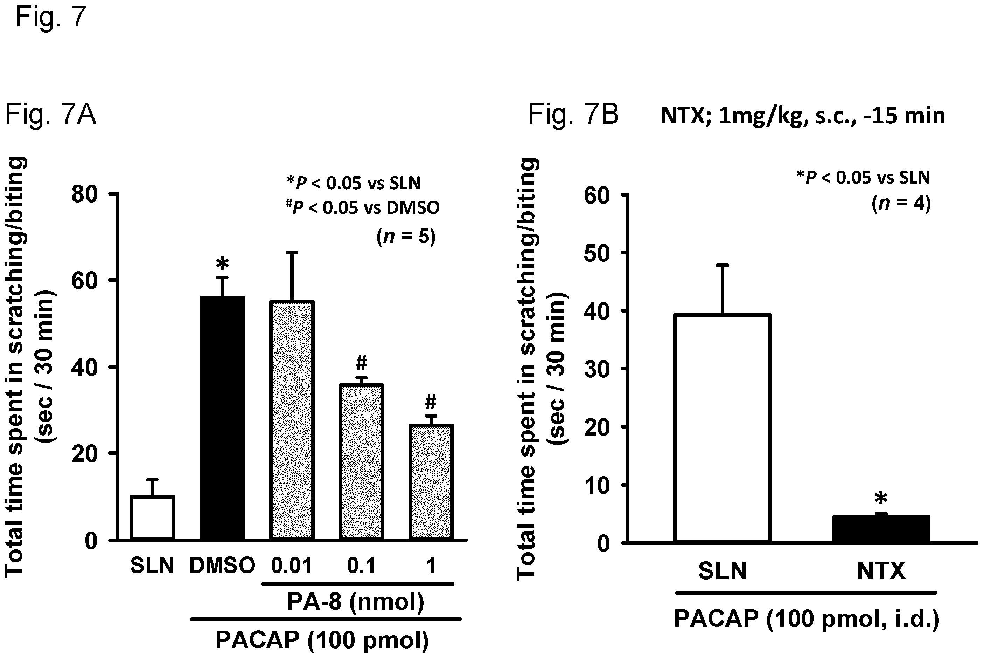

The biting, licking or scratching behavior induced by waist intradermal administration (10 µL) of PACAP (100 pmol) was suppressed in a dose-dependent manner by simultaneous intradermal administration of PA-8 (0.01 to 1 nmol) (Figure 7A). SLN indicates intradermal administration of normal saline. DMSO (10% solution, dissolved in normal saline) is a solvent for PA-8. In addition, when the opioid receptor antagonist, naltrexone (NTX: 1 mg/kg), had been subcutaneously administered 15 minutes before the PACAP intradermal administration, the itch behavior induced by PACAP (100 pmol) intradermal administration was significantly suppressed (Figure 7B). SLN indicates that normal saline is administered instead of NTX.

### [Example 9]

The itch behavior induced by the PACAP intradermal administration is suppressed by simultaneous intradermal administration of PA-8 or the compound 2o with PACAP.

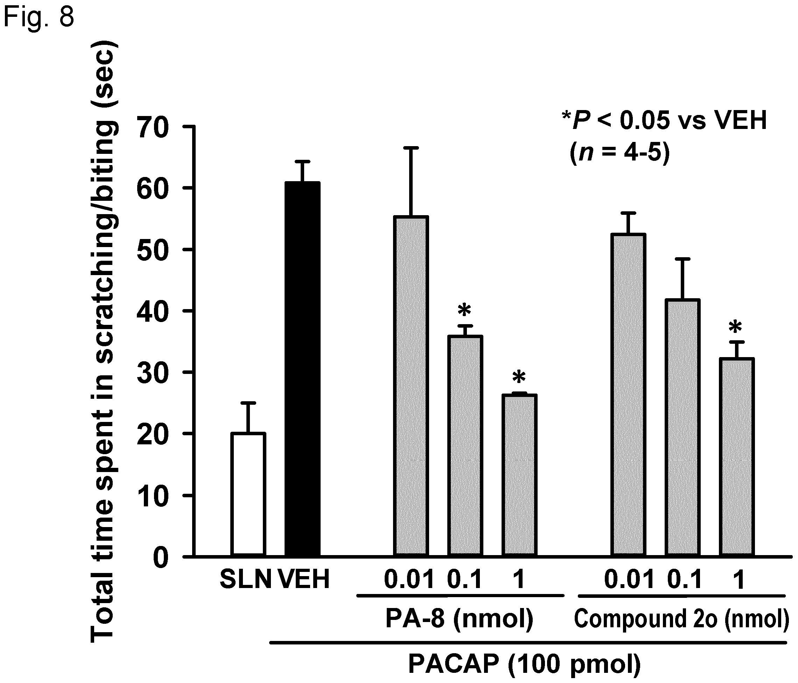

The biting, licking or scratching behavior induced by waist intradermal administration (10 µL) of PACAP (100 pmol) was suppressed in a dose-dependent manner by simultaneous intradermal administration of PA-8 (0.01 to 1 nmol) or the compound 2o (0.01 to 1 nmol) with PACAP (Figure 8). SLN indicates intradermal administration of normal saline. VEH (10% DMSO solution, dissolved in normal saline) is a solvent for PA-8 and the compound 2o.

### [Example 10]

The itch behavior induced by the PACAP intradermal administration is suppressed by simultaneous intradermal administration of PA-9 or the compound 3 with PACAP.

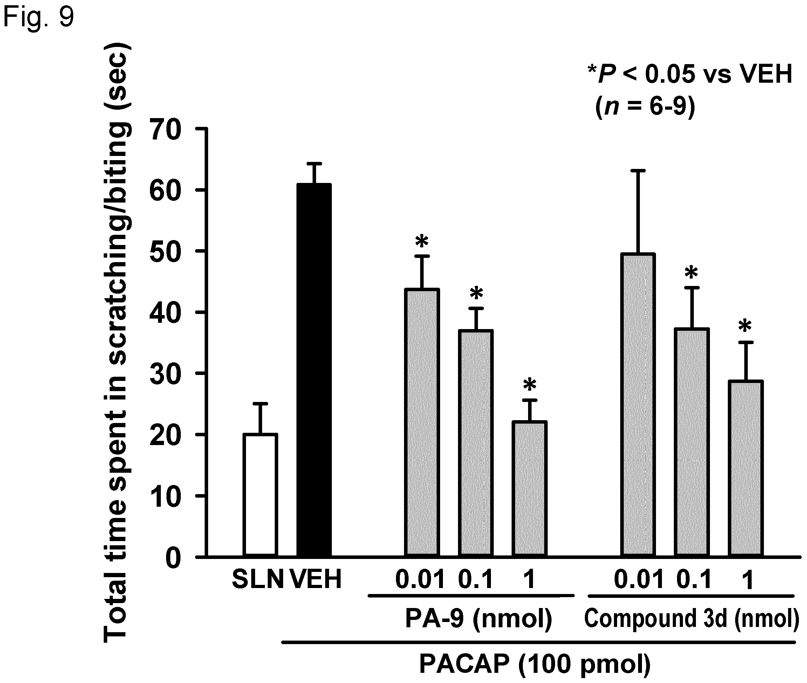

The biting, licking or scratching behavior induced by waist intradermal administration (10 µL) of PACAP (100 pmol) was suppressed in a dose-dependent manner by simultaneous intradermal administration of PA-9 (0.01 to 1 nmol) or the compound 3d (0.01 to 1 nmol) with PACAP (Figure 9). SLN indicates intradermal administration of normal saline. VEH (10% DMSO solution, dissolved in normal saline) is a solvent for PA-9 and the compound 3d.

### [Example 11]

The itch behavior induced by intradermal administration of chloroquine is suppressed by intrathecal pre-administration of PA-8 or PA-9, but is not suppressed by simultaneous intradermal administration of PA-8 or PA-9 with chloroquine.

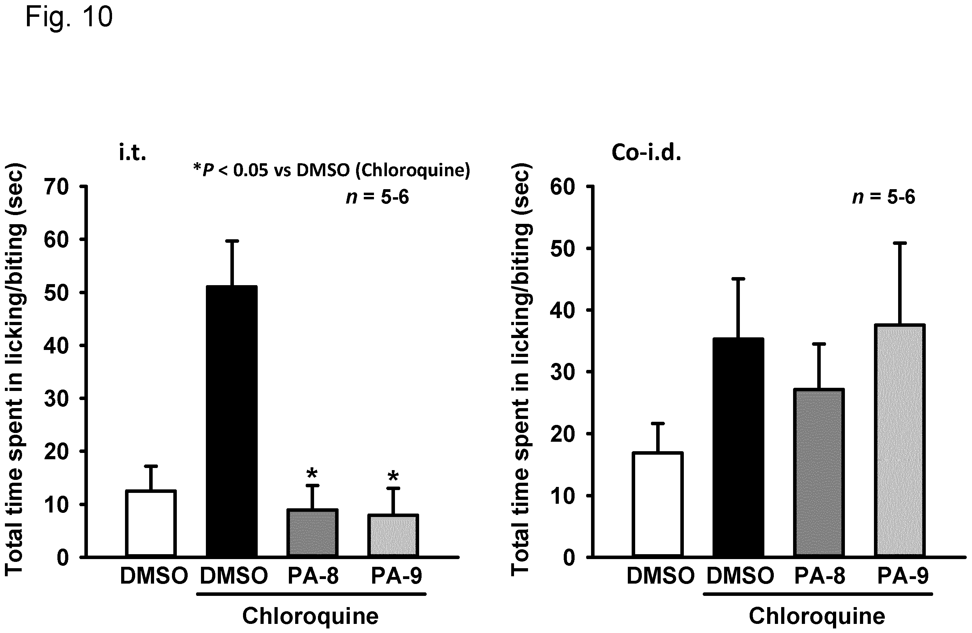

Chloroquine (100 µg/10 µL) was intradermally injected into the waist of each mouse (intradermal injection, i.d.). The reaction of biting, licking or scratching the injected site was defined as itch behavior, and the behavioral time was counted every 5 minutes. As a result, intrathecal pre-administration (i.t., 10 minutes before i.d.) of PA-8 (1 nmol) or PA-9 (1 nmol) almost completely suppressed the chloroquine-induced itch behavior, but such itch behavior was not suppressed by simultaneous intradermal administration (Co-i.d.) of PA-8 or PA-9 with chloroquine (Figure 10). DMSO (10% solution, dissolved in normal saline) is a solvent for PA-8 or PA-9.

### [Example 12]

Itch behavior induced by intradermal administration of Compound 48/80 is suppressed by intrathecal pre-administration of PA-8 or PA-9, but is not suppressed by simultaneous intradermal administration of PA-8 or PA-9 with Compound 48/80.

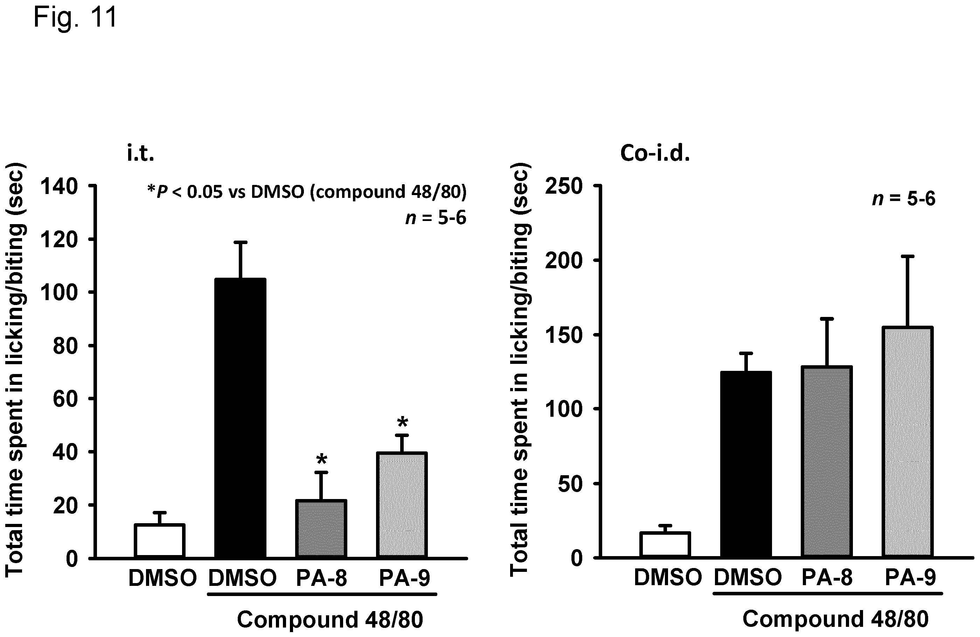

Compound 48/80 (100 µg/10 µL) was intradermally injected into the waist of each mouse (intradermal injection, i.d.). The reaction of biting, licking or scratching the injected site was defined as itch behavior, and the behavioral time was counted every 5 minutes. As a result, intrathecal pre-administration (i.t., 10 minutes before i.d.) of PA-8 (1 nmol) or PA-9 (1 nmol) almost completely suppressed the Compound 48/80-induced itch behavior, but such itch behavior was not suppressed by simultaneous intradermal administration (Co-i.d.) of PA-8 or PA-9 (Figure 11). DMSO (10% solution, dissolved in normal saline) is a solvent for PA-8 or PA-9.

### [Example 13] Drug efficacy evaluation using the atopic dermatitis mouse model

The present test was carried out with reference to the method of Kitamura et al. that is a test system published as a method for evaluating itch in animals (Kitamura A., Takata R., Aizawa S., et al.: A murine model of atopic dermatitis can be generated by painting the dorsal skin with hapten twice 14 days apart, Scientific Rep., 8: 5988, 2018).

Five-week-old ICR mice (30 mice) were each placed in a cage, and were then conditioned for 1 week. Thereafter, three days before initiation of the test, the waist of each mouse was shaved. On the experiment start date, for the purpose of destroying the function of skin barrier, tape stripping was carried out, and 0.15% 2,4-dinitrofluorobenzene (DNFB) solution [acetone/olive oil (3 : 1)] was applied to the shaved waist of each mouse, using a 100-µL pipette. Seven days later, the 0.15% DNFB solution was applied thereto once again, and two hours later, video observation was carried out for 1 hour. The shaved 30 mice were divided into 5 groups (6 mice per group), namely, a 30 mg/kg PA-8 oral (p.o.) administration group, a 30 mg/kg compound 2o oral (p.o.) administration group, a 30 mg/kg PA-9 oral (p.o.) administration group, a 30 mg/kg compound 3d (p.o.) administration group, and a solvent administration group (VEH: oral administration of 10% DMSO aqueous solution). Oral administration was carried out 30 minutes before initiation of the video observation.

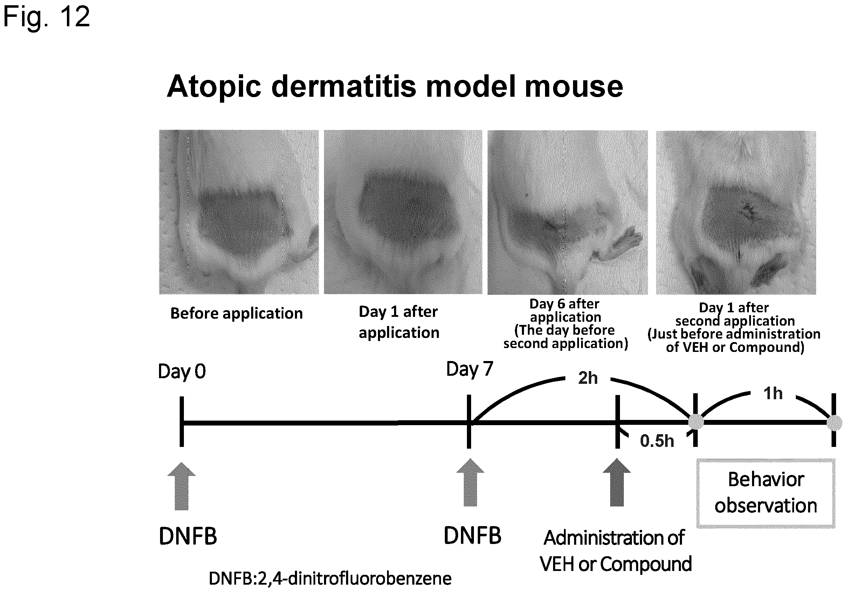

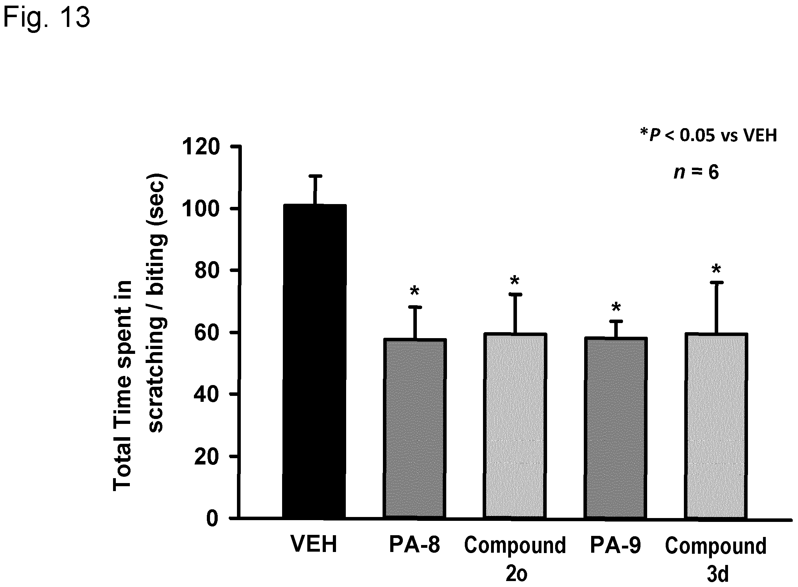

An outline of the procedures of a behavioral experiment using the atopic dermatitis mouse model is shown in Figure 12, and the results obtained by evaluating the time spent in scratching of each group are shown in Figure 13. As shown in Figure 13, it is found that the itch behavior induced by the DNFB treatment was significantly suppressed by oral administration of PA-8, PA-9 or the derivatives thereof (compound 2o and compound 3d).

### [Example 14] Drug efficacy evaluation using the psoriasis mouse model

The present test was carried out with reference to the method of van der Fits et al. that is a test system published as a method for evaluating itch in animals (van der Fits L., Mourits S., Voerman JSA., et al.: Imiquimod-Induced Psoriasis-Like Skin Inflammation in Mice Is Mediated via the IL-23/IL-17 Axis, J. Immunol., 182, 5836-5845, 2009).

Five-week-old ICR mice (20 mice) were each placed in a cage, and were then conditioned for 1 week. Thereafter, three days before initiation of the test, the waist of each mouse was shaved. Then, 62.5 mg of commercially available imiquimod cream (5% Beselna Cream; MOCHIDA PHARMACEUTICAL CO., LTD.) was applied onto the shaved waist skin of the mice once a day for 5 days, so as to produce imiquimod-induced psoriasis model. Twenty-four hours after the final application of the cream (Day 5), video observation was carried out for 1 hour. The shaved 20 mice were divided into 5 groups (4 mice per group), namely, a 30 mg/kg PA-8 oral (p.o.) administration group, a 30 mg/kg compound 2o oral (p.o.) administration group, a 30 mg/kg PA-9 oral (p.o.) administration group, a 30 mg/kg compound 3d (p.o.) administration group, and a solvent administration group (VEH: oral administration of 10% DMSO aqueous solution). Oral administration was carried out 30 minutes before initiation of the video observation.

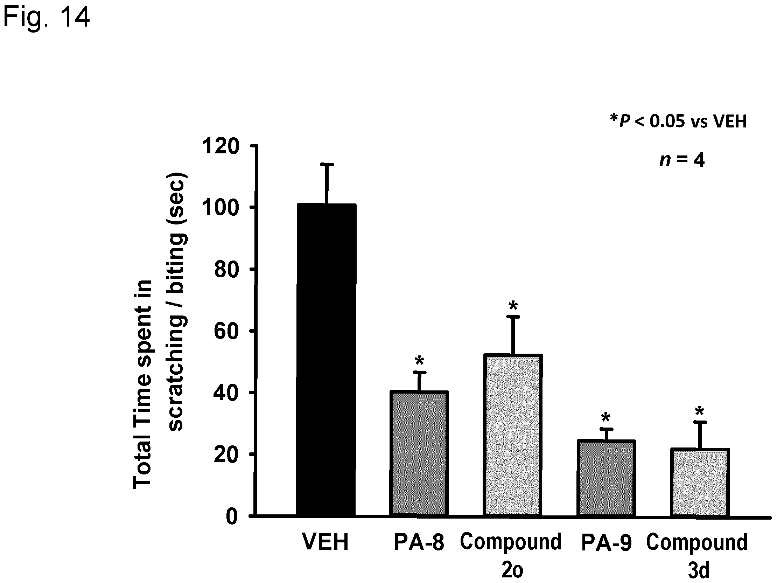

The results obtained by evaluating the time spent in scratching of each group are shown in Figure 14. As shown in Figure 14, it is found that the itch behavior induced by application of imiquimod was significantly suppressed by oral administration of PA-8, PA-9 or the derivatives thereof (compound 2o and compound 3d).

## Claims

1. A composition comprising a compound represented by the following formula (I), 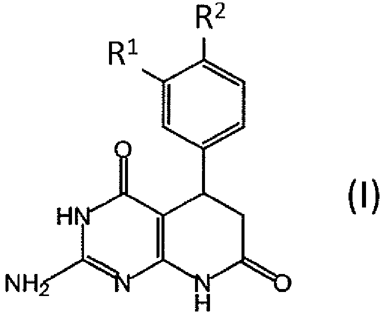
wherein R¹ is a hydrogen atom, a C₁₋₆-alkyl group, a C₁₋₆-alkoxy group, a C₂₋₆-alkenyloxy group, a halogen atom, a C₁₋₆-haloalkyl group, a C₁₋₆-haloalkoxy group, or a substituted or unsubstituted phenyl group; and R² is a hydrogen atom, a C₁₋₆-alkyl group, a C₁₋₆-alkoxy group, a C₂₋₆-alkenyloxy group, a halogen atom, a C₁₋₆-haloalkyl group, a C₁₋₆-haloalkoxy group, or a substituted or unsubstituted phenyl group;
or a salt thereof, or a solvate thereof,
for use as an antipruritic agent.

2. The composition according to Claim 1 for use in treating and/or preventing itch associated with contact dermatitis, atopic dermatitis, dry skin and/or psoriasis.

3. A composition comprising a compound represented by the following formula (II), 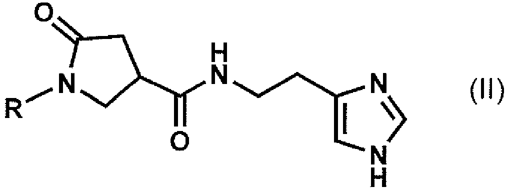
wherein R is an indazolyl group substituted with a halogen atom; a substituted or unsubstituted phenyl group; a pyrazolyl group; or a substituted or unsubstituted aralkyl group;
or a salt thereof, or a solvate thereof,
for use as an antipruritic agent.

4. The composition for use according to Claim 3, wherein R is an indazolyl group substituted with a halogen atom in the formula (II).

5. The composition for use according to Claim 3, wherein R is an indazolyl group substituted with a chlorine atom in the formula (II).

6. The composition according to any one of Claims 3 to 5 for use in treating and/or preventing itch associated with contact dermatitis, atopic dermatitis, dry skin and/or psoriasis.

## Patentansprüche

1. Zusammensetzung, umfassend eine Verbindung, die durch die folgende Formel (I) dargestellt ist 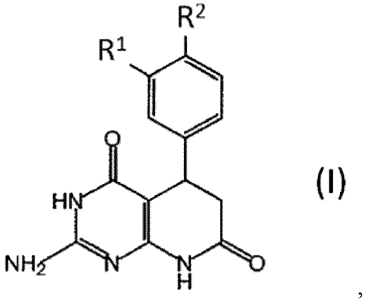
wobei R¹ ein Wasserstoffatom, eine C₁₋₆-Alkylgruppe, eine C₁₋₆-Alkoxygruppe, eine C₂₋₆-Alkenyloxygruppe, ein Halogenatom, eine C₁₋₆-Haloalkylgruppe, eine C₁₋₆-Haloalkoxygruppe oder eine substituierte oder unsubstituierte Phenylgruppe ist; und R² ein Wasserstoffatom, eine C₁₋₆-Alkylgruppe, eine C₁₋₆-Alkoxygruppe, eine C₂₋₆-Alkenyloxygruppe, ein Halogenatom, eine C₁₋₆-Haloalkylgruppe, eine C₁₋₆-Haloalkoxygruppe oder eine substituierte oder unsubstituierte Phenylgruppe ist;
oder ein Salz davon oder ein Solvat davon,
zur Verwendung als einen juckreizstillenden Wirkstoff.

2. Zusammensetzung nach Anspruch 1 zur Verwendung bei der Behandlung und/oder Prävention von Juckreiz im Zusammenhang mit Kontaktdermatitis, atopischer Dermatitis, Hauttrockenheit und/oder Psoriasis.

3. Zusammensetzung, umfassend eine Verbindung, die durch die folgende Formel (II) dargestellt ist 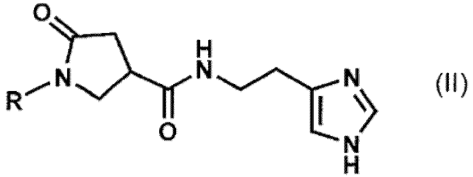
wobei R eine Indazolylgruppe, substituiert mit einem Halogenatom; eine substituierte oder unsubstituierte Phenylgruppe; eine Pyrazolylgruppe; oder eine substituierte oder unsubstituierte Aralkylgruppe ist;
oder ein Salz davon oder ein Solvat davon,
zur Verwendung als einen juckreizstillenden Wirkstoff.

4. Zusammensetzung zur Verwendung nach Anspruch 3, wobei R eine Indazolylgruppe ist, die mit einem Halogenatom in der Formel (II) substituiert ist.

5. Zusammensetzung zur Verwendung nach Anspruch 3, wobei R eine Indazolylgruppe ist, die mit einem Chloratom in der Formel (II) substituiert ist.

6. Zusammensetzung nach einem der Ansprüche 3 bis 5 zur Verwendung bei der Behandlung und/oder Prävention von Juckreiz im Zusammenhang mit Kontaktdermatitis, atopischer Dermatitis, Hauttrockenheit und/oder Psoriasis.

## Revendications

1. Composition, comprenant un composé représenté par la formule suivante (I), 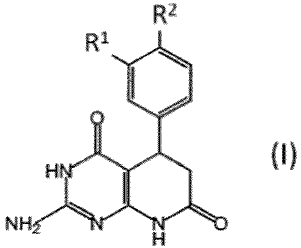
dans laquelle R¹ est un atome d'hydrogène, un groupe C₁₋₆-alkyle, un groupe C₁₋₆-alkoxy, un groupe C₂₋₆-alcényloxy, un atome d'halogène, un groupe C₁₋₆-haloalkyle, un groupe C₁₋₆-haloalkoxy, ou un groupe phényle substitué ou non substitué ; et R² est un atome d'hydrogène, un groupe C₁₋₆-alkyle, un groupe C₁₋₆-alkoxy, un groupe C₂₋₆-alcényloxy, un atome d'halogène, un groupe C₁₋₆-haloalkyle, un groupe Cₗ₁₋₆-haloalkoxy, ou un groupe phényle substitué ou non substitué ;
ou un sel de celui-ci, ou un solvate de celui-ci,
pour utilisation en tant qu'agent antiprurigineux.

2. Composition selon la revendication 1, pour utilisation dans le traitement et/ou la prévention de démangeaison associée la dermatite de contact, à la dermatite atopique, à la peau sèche, et/ou au psoriasis.

3. Composition, comprenant un composé représenté par la formule suivante (II), 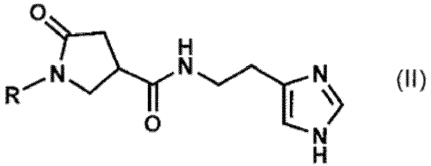
dans laquelle R est un groupe indazolyle substitué avec un atome d'halogène ; un groupe phényle substitué ou non substitué ; un groupe pyrazolyle ; ou un groupe aralkyle substitué ou non substitué ;
ou un sel de celui-ci, ou un solvate de celui-ci,
pour utilisation en tant qu'agent antiprurigineux.

4. Composition pour utilisation selon la revendication 3, dans laquelle R est un groupe indazolyle substitué avec un atome d'halogène dans la formule (II).

5. Composition pour utilisation selon la revendication 3, dans laquelle R est un groupe indazolyle substitué avec un atome de chlore dans la formule (II).

6. Composition selon l'une quelconque des revendications 3 à 5 pour utilisation dans le traitement et/ou la prévention de démangeaison associée la dermatite de contact, à la dermatite atopique, à la peau sèche, et/ou au psoriasis.
